(19) Europäisches Patentamt
European Patent Office
Office européen des brevets

(11) EP 3 722 422 B1

(12) EUROPEAN PATENT SPECIFICATION

(45) Date of publication and mention of the grant of the patent:
**06.07.2022 Bulletin 2022/27**

(21) Application number: **18898516.2**

(22) Date of filing: **30.12.2018**

(51) International Patent Classification (IPC):
***C12N 15/10** (2006.01)* ***C12N 9/22** (2006.01)*

(52) Cooperative Patent Classification (CPC):
(C-Sets available)
**C12N 15/1006; C12N 9/22; C12N 11/14; C12N 15/1013;** C07K 2319/20; C07K 2319/21; C07K 2319/30; C07K 2319/43; C07K 2319/70

(Cont.)

(86) International application number:
**PCT/CN2018/125973**

(87) International publication number:
**WO 2019/134630 (11.07.2019 Gazette 2019/28)**

(54) **METHOD FOR ISOLATING DNA BY USING CAS PROTEIN SYSTEM**

VERFAHREN ZUR ISOLIERUNG VON DNA UNTER VERWENDUNG EINES CAS-PROTEIN-SYSTEMS

PROCÉDÉ POUR ISOLER DE L'ADN À L'AIDE D'UN SYSTÈME DE PROTÉINE CAS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.01.2018 CN 201810001073**

(43) Date of publication of application:
**14.10.2020 Bulletin 2020/42**

(73) Proprietor: **Cure Genetics Co., Ltd**
**Suzhou, Jiangsu 215123 (CN)**

(72) Inventors:
- **WU, Yao**
**Suzhou, Jiangsu 215123 (CN)**
- **WANG, Xingxing**
**Suzhou, Jiangsu 215123 (CN)**
- **LI, Qiushi**
**Suzhou, Jiangsu 215123 (CN)**

(74) Representative: **Berger, Axel Bernhard**
**Bardehle Pagenberg Partnerschaft mbB**
**Patentanwälte, Rechtsanwälte**
**Prinzregentenplatz 7**
**81675 München (DE)**

(56) References cited:
**CN-A- 106 978 428** **CN-A- 107 406 875**
**US-A1- 2014 356 867**

- **LIU XIN ET AL: "In SituCapture of Chromatin Interactions by Biotinylated dCas9", CELL, ELSEVIER, AMSTERDAM NL, vol. 170, no. 5, 25 August 2017 (2017-08-25), page 1028, XP085170527, ISSN: 0092-8674, DOI: 10.1016/J.CELL.2017.08.003**
- **ZACHARY J WALDRIP ET AL: "A CRISPR-based approach for proteomic analysis of a single genomic locus", EPIGENETICS, vol. 9, no. 9, 18 July 2014 (2014-07-18), pages 1207-1211, XP055771766, US ISSN: 1559-2294, DOI: 10.4161/epi.29919**
- **FUJITA TOSHITSUGU ET AL: "Efficient isolation of specific genomic regions and identification of associated proteins by engineered DNA-binding molecule-mediated chromatin immunoprecipitation (enChIP) using CRISPR", BIOCHEMICAL AND BIOPHYSICAL RESEARCH COMMUNICATIONS, ELSEVIER, AMSTERDAM NL, vol. 439, no. 1, 11 August 2013 (2013-08-11), pages 132-136, XP028715147, ISSN: 0006-291X, DOI: 10.1016/J.BBRC.2013.08.013**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 722 422 B1

- **FUJITA, T. et al.: "Efficient Sequence-specific Isolation of DNA Fragments and Chromatin by in Vitro enChIP Technology Using Recombinant CRISPR Ribonucleoproteins", Genes to Cells, vol. 21, no. 4, 31 December 2016 (2016-12-31), pages 370-377, XP055621734,**

(52) Cooperative Patent Classification (CPC): (Cont.)

C-Sets
**C12N 15/1006, C12Q 2521/301;**
**C12N 15/1013, C12Q 2521/301**

## Description

### Technical Field

The present invention relates to a method for isolating DNA, and relates in particular to a method for isolating DNA from a solution by using a Cas protein in the absence of RNA mediation.

### Background Art

Under normal circumstances, the vast majority of deoxyribonucleic acids (DNA) are located in cells. However, fragments of DNA are often found outside cells, regardless of whether they are cells of microorganisms, plants or animals. Such nucleic acids are collectively referred to as cell-free DNA. The discovery of cell-free DNA in peripheral blood was reported, for the first time, by Mandel and Métais in 1948 (Mandel P., C R Hebd Seances Acad Sci (Paris), 1948, 142 (3): 241-3). After half a century of research, scientists have found that both healthy and diseased populations have a small portion of DNA which is free of cells. Such DNA is usually found in blood, plasma, serum or other body fluids. Extracellular DNA found in various samples is not easily degraded because it is protected by nucleases (for example, they are secreted in the form of protein-lipid complexes, often bound to proteins, or encapsulated in vesicles). The content of such extracellular DNA, in populations having diseases such as malignant tumors and infectious diseases, is often much higher than that in normal people, and therefore, has great application prospects in disease screening, diagnosis, prognosis, disease progression monitoring, and even in the identification of therapeutic targets. In addition, maternal blood is often accompanied by a considerable amount of fetal DNA, which can be used for a variety of detections, such as gender identification, test and evaluation of chromosomal abnormality, and can also be used for monitoring pregnancy-related complications. Just because of the strong clinical diagnostic relevance, cell-free DNA has a huge application potential in noninvasive diagnosis and prognosis, and can be used in, for example, noninvasive prenatal genetic detection, tumor detection, transplantation medicine and the detections of many other diseases.

At present, many methods for extracting and analyzing single-stranded or double-stranded cell-free DNA from various biological fluids have been reported, including an anion exchange method (WO 2016198571 A1), a silica bead extraction method (WO 2015120445 A1), and a Triton/heating/phenol method (Xue et al. Clinica Chimica Acta 2009. 404:100-104), etc. These methods usually use a chaotropic agent to release the cell-free DNA from the encapsulation of proteins, and then isolate the cell-free DNA by means of physical adsorption or precipitation. However, the yield of DNA extracted by using these methods is very low and it is difficult to extract small amounts of DNA from large samples. For example, at present, the commercially available QIAamp® serum/plasma nucleic acid purification kit (Circulating Nucleic Acid kit, QIAGEN Inc.) for cell-free nucleic acid extraction has a cell-free DNA extraction efficiency of less than 50%.

The clustered regularly interspaced short palindromic repeats (CRISPR) and CRISPR binding protein (Cas protein) form a set of a powerful nuclease system capable of cleaving almost all genomic sequences adjacent to the protospacer-adjacent motif (PAM) in eukaryotic cells (Cong et al. Science 2013. 339: 819-823). So far, all applications related to the CRISPR/Cas system further comprise, in addition to the Cas protein, an RNA component. The RNA component is a double-stranded guide RNA structure composed of a CRISPR RNA (crRNA) and a transactivated crRNA (tracrRNA), which directs the Cas protein to cleave DNA sites complementary to the crRNA sequence (Jinek et al. Science 2012. 337: 816-821). In order to further simplify the CRISPR/Cas9 system, researchers have used engineering methods to join parts of the two components of the double-stranded guide RNA (crRNA and tracrRNA) into a chimeric single-stranded guide RNA (sgRNA). This embodies the powerful gene recognition and gene editing capabilities of the CRISPR/Cas9 system, however, the nature of the Cas protein itself has not been studied thoroughly. So far, no researchers have discovered the potential of the Cas protein and a variant thereof as a nuclease alone for recognizing and binding to DNA.

The scientific article by Liu Xin et al., entitled "In Situ Capture of Chromatin Interactions by Biotinylated dCas9", CELL, ELSEVIER, AMSTERDAM NL,vol.170, no. 5,25 August 2017 (2017-08-25), page 1028, XP085170527, ISSN: 0092-8674, DOI: 10.1016/J.CELL.2017.08.003 describes a CRISPR affinitiy purification in situ of regulatory elements (CAPTURE) using an in vivo biotinylated nuclease-deficient Cas9 protein and sequence-specific guide RNAs.

The scientific article by Zachary Waldrip et al., entitled "A CRISPR-based approach for proteomic analysis of a single genomic locus", EPIGENETICS, vol.9, no. 9, 18 July 2014 (2014-07-18), pages 1207-1211, XP055771766, US ISSN: 1559-2294, DOI: 10.4161 /epi.29919 is directed to the use of the Cas9 and guide RNA (gRNA) components of the CRISPR system for gRNA-directed purification of a discrete section of chromatin, the so-called CRISPR-based Chromatin Affinity Purification with Mass Spectrometry (CRISP-ChAP-MS) approach.

US 2014/356867 A1 relates to nucleic acid enrichment using a Cas9-gRNA complex comprising a Cas9 protein and a guide RNA (gRNA).

The scientific article by Toshitsugu Fujita et al., entitled "Efficient isolation of specific genomic regions and identification of associated proteins by engineered DNA-binding molecule-mediated chromatin immunoprecipitation (enChIP) using CRISPR", BIOCHEMICAL AND BIOPHYSICAL RESEARCH COMMUNICATIONS, ELSEVIER, AMSTERDAMNL,vol. 439, no. 1, 11 August 2013 (2013-08-11), pages 132-136, XP028715147, ISSN: 0006-291X, DOI: 10.1016/J. BBRC.2013.08.013 describes a method for purification of specific genomic regions using engineered DNA-binding molecule-mediated chromatin immunoprecipitation (enCHIP) with a catalytically inactive form of Cas9 (dCas9) which is co-expressed with a guide RNA (gRNA).

At present, there is a strong demand on the market for an efficient method for extracting cell-free DNA in order to efficiently extract the cell-free DNA from large samples of various biological fluids for subsequent analysis and detection.

## Summary of the Invention

In order to improve the extraction efficiency of DNA in a solution, the present invention provides for a method for isolating DNA from a solution by using a Cas protein in the absence of RNA mediation, comprising the steps of a) mixing a DNA solution with a Cas protein system; and c) isolating DNA from a complex formed by the binding of the Cas protein system with DNA.

Under the condition of the same content of DNA samples, the extraction method of the present invention eliminates cumbersome sample processing processes, reduces requirements for sample usage amounts, and greatly increases the efficiency of extracting cell-free DNA.

## Brief Description of the Drawings

Figure 1: expression vector pSMART-his-S.P.dCas9-FLAG containing the his-S.P.dCas9-FLAG gene fragment.
Figure 2: electrophoresis band of cell-free DNA (SEQ ID NO. 8) of 486 bp in length bound to a dCas protein.

Lane 1 is the band of cell-free DNA itself.

Lane 2 shows the situation after the dCas9 and cell-free DNA (SEQ ID NO. 8) are bound.

## Detailed Description of The Invention

<u>Cas protein</u>

Clustered regularly interspaced short palindromic repeats (CRISPR) and the CRISPR binding protein (Cas protein) form a powerful nuclease system. The CAS protein can be divided into four different functional modules: a target recognition module (interval acquisition); an expression module (crRNA processing and target combination); an interference module (target cleaving); and an auxiliary module (supervision and other CRISPR-related functions). In recent years, a large amount of structural and functional information has been accumulated for core Cas proteins (Cas1-Cas10), which allows them to be classified into these modules. The CRISPR-related endonuclease Cas protein can target specific genomic sequences through the single-stranded guide RNA (sgRNA). Taking the *Streptococcus pyogenes* Cas9 protein (spCas9) as an example, the protein comprises a double-leaf structure composed of a target recognition module and a nuclease module (interference module), which accommodates the sgRNA-DNA heteroduplex in the positivelycharged groove at the interface. Moreover, the target recognition region is necessary for binding sgRNA and DNA. The nuclease region comprises HNH and RuvC nuclease domains, which are suitable for cleaving the complementary and noncomplementary strands of the target DNA, respectively. The nuclease region also contains a carboxyl-terminal domain responsible for interaction with the protospacer-adjacent motif (PAM). However, to date, there is no report from any research group about the structural region where the Cas protein directly binds to DNA, or the crystal structure of the Cas-DNA complex.

Therefore, in the present invention, in the absence of RNA mediation, the Cas protein itself is a nucleic acid binding system. Under normal circumstances, the Cas protein can recognize and bind to almost all DNA sequences without RNA mediation, and does not cleave the bound DNA sequences.

The Cas protein of the present invention is a Cas protein with the DNA binding function, such as a natural Cas protein, a mutant Cas protein, a mutant Cas protein which loses the gRNA binding ability, and a mutant Cas protein which loses the nuclease activity (dead Cas, dCas).

Non-limiting examples of Cas proteins include: Cas1, Cas1B, Cas2, Cas3, Cas4, Cas5, Cas6, Cas7, Cas8, Cas9 (also known as Csn1 or Csx12), Cas10, Cas12, Cas13, Cas14, Csy1, Csy2, Csy3, Cse1, Cse2, Cscl, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, and homologous proteins thereof in different species, endonuclease- inactivated mutant proteins or modified forms thereof.

In a specific embodiment, the Cas protein of the present invention is a Cas9 protein, more preferably a mutant Cas9 protein which loses nuclease activity (dead Cas9, dCas9)

Cas9, also known as Csn1 or Csx12, is a giant protein that participates in both crRNA biosynthesis and destruction of invading DNA. Cas9 has been described in different bacterial species such as *S. thermophiles, Listeria innocua* (Gasiunas, Barrangou et al. 2012; Jinek, Chylinski et al. 2012) and *S. Pyogenes* (Deltcheva, Chylinski et al. 2011). Cas9 of the present invention includes, but is not limited to, the following types: *Streptococcus pyogenes* Cas9 protein, and its amino acid sequence is under SwissProt database accession number Q99ZW2; *Neisseria meningitides* Cas9 protein, and its amino acid sequence is under UniProt database number A1IQ68; *Streptococcus thermophilus* Cas9 protein, and its amino acid sequence is under UniProt database number Q03LF7; and *Staphylococcus aureus* Cas9 protein, and its amino acid sequence is under UniProt database number J7RUA5.

The Cas protein that acts on the double-stranded DNA generally has two active sites for endonuclease. If there is and only one site is mutated or deleted to cause the enzyme activity at this site to be inactivated, a nickase that cleaves a single strand of DNA can be obtained, such as Cas9-nickase is widely used because of its high fidelity and the characteristics of cleaving a single strand of DNA.

In addition, screening for the Cas protein variant with one or more point mutations by mutation has been proved to improve its specificity, reduce the off-target rate of genome editing, or make it compatible with more diverse PAM sequences, such as eSpCas9, SpCas9-HF, HeFSpCas9, HIFI-SpCas9 and xCas9 (Christopher A. V et al. Nature Medicine 2018 (24), pp. 1216-1224; Johnny H. H et al. Nature 2018 556 (7699), pp. 57-63).

In view of the Cas protein comprising multiple functional modules, the protein sequence is long, in order to facilitate packaging and transportation and function control, the protein sequence will also be truncated through protein engineering research to form a Cas protein system, such as a Split-SpCas9 system comprising complexes formed by truncated proteins (Jana M et al. Plant Biotechnology Journal 2017 15, pp. 917-926).

In some embodiments, the Cas protein is a fusion protein or protein complex containing the above protein or a mutant thereof, including but not limited to variants linked to other amino acid sequences on the Cas. The fusion of other functional proteins on the basis of the above Cas protein or its variants can increase the specificity and effectiveness of the Cas protein function, and can also produce effects on the genome other than cleavage. For example, the fusion of a FokI on the Cas or dCas can increase the specificity of the Cas9 protein for genomic cleavage, because only the FokI dimerization has a cleaving activity, which requires a pair of recognition regions to complete the cleavage and reduce the off-target rate. For another example, a FokI is connected to the Cas protein in which a part of the functional domains is truncated but the DNA binding ability thereof is preserved (Ma et al. ACS Synth. Biol., 2018, 7 (4), pp 978-985). For another example, a basemodifying enzyme (such as deaminase, cytosine deaminase and adenine deaminase) is fused to the Cas-nickase or dCas, which may efficiently perform targeted base modification on the target region of the genome (Komor et al. Sci. Adv. 2017.3: eaao4774). For another example, the dCas9 is fused with some protein domains that may regulate the gene expression, which may effectively regulate the expression of target genes. For example, the dCas9 fused with transcriptional activators, such as VP64, VPR and the like, may bind near the target gene to activate the expression guided by gRNA; conversely, the dCas9 fused with a transcriptional repressor (such as SRDX) will downregulate the target gene. dSpCas9-Tet1 and dSpCas9-Dnmt3a can be used to modify the epigenetic state, and regulate the methylation state of the endogenous gene promoter to regulate the protein expression.

Cas protein with the DNA binding function

The Cas protein with the DNA binding function may be a natural Cas protein, or a mutation made outside the DNA-binding functional region of the Cas protein, or a Cas protein obtained after mutation in the DNA-binding functional region.

In order to detect whether the Cas protein has a DNA binding function, gel electrophoresis may be used to compare the gel band positions of the protein-bound DNA and the protein-unbound DNA for discrimination (for the detection method, see example 1).

Nuclease-inactivated mutant Cas protein (dCas protein)

The nuclease-inactivated mutant Cas protein (dCas protein) is a variant obtained by mutation of the Cas protein, and its endonuclease activity is inactivated or substantially inactivated, causing the Cas protein to lose or substantially lose the endonuclease activity, and thus fail to cleave the target sequence. The above non-limiting examples of the Cas proteins can be transformed into the dCas proteins by endonuclease-inactivating mutations, including insertion, deletion, or substitution of one or more amino acid residues.

For example, certain Cas9 mutations may cause the Cas9 protein to lose or substantially lose endonuclease activity, and thus fail to cleave the target sequence. For the Cas9 of a certain species, such as spCas9, exemplary mutations that reduce or eliminate the endonuclease activity include one or more mutations in the following positions: D10, G12, G17, E762, H840, N854, N863, H982, H983, A984, D986 or A987. The literature proves that guide RNA (gRNA)-mediated endonuclease inactivation mutation Cas9 (referred to as dCas9) can lead to the suppression of the expression of *E. coli*-specific endogenous genes and EGFP reporter genes in human cells (Qi et al. Cell 2013. 152:1173-1183). This study proves that the use of gRNA-mediated dCas9 technology may accurately recognize and bind to the corresponding genome.

The dCas protein may be a non-naturally occurring protein in nature, and is a variant obtained by protein engineering or by random mutagenesis, of which the endonuclease activity is inactivated or substantially inactivated. For example, the corresponding dCas9 protein can be obtained by mutation, that is, the deletion or insertion or substitution of at least one residue in the amino acid sequence of the *Streptococcus pyogenes* Cas9 endonuclease.

In some embodiments, the dCas9 protein may be a variant obtained by mutations inactivating or substantially inactivating the endonuclease activity of Cas9 of different species, including but not limited to, *Streptococcus pyogenes* Cas9 protein, *Neisseria meningitides* Cas9 protein, *Streptococcus thermophilus* Cas9 protein, *Staphylococcus aureus* Cas9 protein, *Streptococcus pneumoniae* Cas9 protein, etc.

## Linker sequence and affinity molecule

The linker sequence of the present invention refers to a molecule directly or indirectly connected to the C-terminus or N-terminus of the Cas protein, which ultimately forms a fusion protein with the Cas protein.

The affinity molecule of the present invention is a molecule coupled to a solid substance and may form a specific binding with the above linker sequence on the Cas protein.

The linker sequence and the affinity molecule of the present invention have a relationship of receptor and ligand to each other. Any protein linker sequence can be used in the practice of the present invention, as long as it can specifically bind to the affinity molecule. The linker sequence and the affinity molecule are selected from one of the following combinations of ligand and receptor: enzyme and substrate, antigen and antibody, and biotin and avidin. The enzyme and substrate are selected from the group of: glutathione transferase and glutathione. The antigen and antibody are selected from the group of: a protein A or a fragment thereof which retains the Fc region-binding function and an immunoglobulin or Fc or Fab fragments thereof, a protein G or a fragment thereof which retains the Fc region-binding function and an immunoglobulin or Fc or Fab fragments thereof, a histidine tag and an anti-histidine tag, a polyhistidine tag and an anti-polyhistidine tag, and a FLAG tag (amino acid sequence: ATLAAAAL) and an anti-FLAG tag. The biotin and avidin are selected from the group of: biotin and avidin (UniProt database number P02701) or streptavidin (UniProt database number P22629), biotin-linked biotin receptor protein (such as AviTag, amino acid sequence: GLNDIFEAQKIEWHE) and avidin or streptavidin, and Strep-tag (amino acid sequence: WSHPQFEK) and avidin or streptavidin.

In the present invention, the linker sequence and the affinity molecule are specifically bound by affinity, and are interchangeable. For example, if the linker sequence is a receptor, the affinity molecule is a ligand that specifically binds to the receptor; and the linker sequence may also be a ligand, so then the affinity molecule is a receptor that specifically binds to the ligand. For another example, if the linker sequence is streptavidin, the affinity molecule is biotin; and the linker sequence may also be biotin, so then the affinity molecule is streptavidin.

## Solid support

The solid substance of the present invention is a solid substance capable of forming a chemical bond with an amino acid residue, and thus can be coupled with an affinity molecule or a Cas protein through a chemical bond.

In terms of materials, suitable solid supports may be solid substances made of gel materials, magnetic materials, cellulose materials, silica gel materials, glass materials or artificial high-molecular polymers. The gel material may be a hydrogel, organic gel, xerogel or nano-composite hydrogel. The magnetic material may be a metal oxide (such as an oxide of iron, cobalt or nickel) wrapped by a polymer material. The cellulose material may be cellulose, cellulose acetate or nitrocellulose. The silica gel material may be a silica support or an organic silica gel support. The artificial high-molecular polymer may be nylon, polyester, polyethersulfone, polyolefin, poly(1,1-vinylidene fluoride), and combinations thereof. In terms of forms, the solid supports may be in forms of beads, chemical film-like columns, glass plates, 6-well/24-well/48-well/96-well/384-well plates, PCR tubes and the like.

After binding the DNA-bound Cas protein to a solid support, such as a chemical film-like column, glass plate, 6-well/24-well/48-well/96-well/384-well plate or PCR tube, DNA is eluted by an eluent.

After binding the DNA-bound Cas protein to a bead-shaped solid support, DNA is eluted by an eluent after enrichment, such as centrifugal enrichment or magnetic enrichment.

The solid supports of the present invention are preferably bead-shaped, such as magnetic beads, gel beads and/or silica gel beads. Non-limiting examples of the gel beads that can be used include: Anti-6X His tag® antibody (agarose gel) (Abcam), Anti-6xHis AlphaLISA® Acceptor beads (PerkinElmer), Dynabeads™ His-Tag Isolation and Pulldown (ThermoFisher), Anti-His-tag mAb-Magnetic Beads (MBL Life science) and His Tag Antibody Plate (GenScript).

Coupling

Conventional methods can be used to couple an affinity molecule or Cas protein to a solid support. For linking protein A and protein G to a solid support, see, for example, Hermanson et al. 1992, Immobilized Affinity Ligand Techniques, Academic Press. Typically, the solid support is activated with a reactive functional group ("activating group"), such as epoxide (epichlorohydrin), cyanide (cyanogen bromide, CNBr), N, N-disuccinimidyl carbonate (DSC), aldehyde or activated carboxylic acid (e.g., N-hydroxysuccinimide (NHS) ester or carbonyldiimidazole (CDI) activated ester). These activating groups can be directly attached to a solid support, such as CNBr, or they can also be part of a "linker" or spacer molecule, typically a linear chain of carbon, oxygen, and nitrogen atoms, as a ten-membered chain of carbon and oxygen found in the linker butanediol diglycidyl ether (a commonly used epoxide coupling agent). Under coupling conditions, the activated solid support is then equilibrated with the Cas protein or affinity molecule. After the coupling reaction is completed, the medium is washed thoroughly.

Cas fusion protein

The Cas fusion protein of the present invention is a fusion protein formed by adding a linker sequence to the N-terminus or C-terminus of the Cas protein or any part of its protein sequence. The fusion Cas protein of the present invention can express the Cas protein and the linker sequence together by genetic engineering methods.

Cas protein system

The Cas protein system of the present invention is a complex containing the Cas protein, which is used to bind to DNA in a solution.

In a specific embodiment, the Cas protein system is a complex formed by coupling a Cas protein to a solid support through a chemical covalent bond.

In a specific embodiment, the Cas protein system is a Cas fusion protein formed by a Cas protein and one or more linker sequences.

In another specific embodiment, the Cas protein system is a complex formed by binding a Cas fusion protein to an affinity molecule coupled to a solid substance.

Binding of the Cas fusion protein to the solid substance coupled with an affinity molecule

A Cas protein and a linker sequence together form a Cas fusion protein. The linker sequence may be directly or indirectly located at the N-terminus or C-terminus of the Cas protein. Taking the solid substance as a gel bead as an example, in a preferred embodiment, when using a gel bead coupled with an affinity molecule to bind the Cas fusion protein, for every 10 μl of gel beads, the initial concentration of Cas fusion protein in the reaction solution is 0.001-20 μg/μl, and preferably 0.01-10 μg/μl of Cas9 fusion protein before adding the gel beads.

The binding of the Cas fusion protein to the solid substance is performed at 4-37°C, and preferably 4°C or room temperature (20-25°C). The binding reaction solution can be deionized water, 1x phosphate buffered saline (PBS), Tris buffer (50 mM Tris-HCl, 150 mM NaCl, pH7.4), or other buffers with a salt ion concentration below 150 mM, free of oil detergents and denaturants, and with an approximately neutral pH. The reaction time is from 30 minutes to overnight, and preferably overnight.

The Cas protein may be directly bound to the solid substance coupled with an affinity molecule without a connexin.

Binding of the Cas protein to cell-free DNA

The Cas protein of the present invention may bind to any DNA in a cell-free state. The DNA may be double- or single-stranded DNA. Cell-free DNA bound to the Cas protein can be isolated from the liquid phase by binding a solid substance to the Cas protein.

DNA bound to the Cas protein of the present invention may be any cell-free DNA. For example, DNA fragment with any sequence obtained by PCR using a genome as a template, DNA fragments with sequences artificially designed, cell-free DNA after cell lysis, genome obtained after cell lysis, and cell-free DNA in human or animal body fluids. In some embodiments, DNA refers to DNA fragments obtained by PCR using a genome as a template, DNA fragments with sequence artificially designed, or cell-free DNA in human or animal body fluids.

As a preferred embodiment, when the Cas protein or Cas fusion protein binds to cell-free DNA, the protein concentration in the DNA binding reaction solution is 0.001-20 μg/μl, and preferably, 0.01-10 μg/μl of the Cas protein or a variant thereof or the Cas fusion protein. In a preferred embodiment, the reaction is performed from room temperature (20-25°C) to 37°C. The DNA binding reaction solution can be deionized water, 1x phosphate buffered saline (PBS), Tris buffer (50 mM Tris-HCl, 150 mM NaCl, pH7.4), or other buffers with a salt ion concentration below 150 mM, free of oil detergents and denaturants, and with a neutral pH, and preferably, 50 mM KCl, 10 mM EDTA, 30 mM Tris-HCl, 0.2% Triton X-100, and 12% glycerol. The reaction time is from 5 minutes to 6 hours, and in one embodiment, the preferred time is from 5 minutes to 2 hours. In another embodiment, the preferred time is from 15 minutes to 1 hour.

Method for isolating cell-free DNA from a solution

The method for isolating cell-free DNA by using the Cas protein of the present invention may be used in three ways:

Method 1: the Cas protein or Cas fusion protein is first bound to cell-free DNA in the DNA binding reaction solution, and then in the protein binding reaction solution, the Cas protein or Cas fusion protein is bound by the solid substance coupled with an affinity molecule. Firstly, the Cas protein or Cas fusion protein and cell-free DNA are incubated and bound in the DNA binding reaction solution under the DNA binding reaction conditions described above, the preferred time is from 5 minutes to 6 hours; and in another embodiment, the preferred time is from 15 minutes to 1 hour. The reaction temperature is from room temperature (20-25°C) to 37°C; after the DNA binding reaction is completed, the solid substance coupled with an affinity molecule is directly added to the solution. Under the protein binding reaction conditions described above, the Cas protein or Cas fusion protein is bound to the solid substance coupled with an affinity molecule. The preferred reaction time is from 30 minutes to overnight, and the reaction temperature is from 4°C to 37°C. Finally, the solid substance is separated by centrifugation and the supernatant is discarded. After the solid substance is washed several times, the Cas protein or a variant thereof or Cas fusion protein, and bound DNA are eluted from the solid substance to obtain an elution solution. The eluent that can be used is a 0.5-10 mg/ml proteinase K solution, 1-5 M NaCl or KCl solution, alkaline eluent (0-0.3 M Tris, 0-0.5 M NaCl, pH 11- 12.5), acid eluent (0-0.3 M glycine HCl, pH 2.5-3.5), competitive eluent (DYKDDDK or FLAG amino acid in TBS, amino acid concentration can be 100 to 500 μg/ml), or PAGE gel sample solution (0.01 M Tris-HCl, 10% glycerol, 0.016% bromophenol blue).

Method 2: the solid substance coupled with an affinity molecule is first bound to the Cas protein or a variant thereof or Cas fusion protein, and then is incubated and bound to cell-free DNA in the DNA binding reaction solution. The reaction conditions, solutions and elution conditions used are the same as those in Method 1.

Method 3: the solid substance coupled with an affinity molecule, the Cas9 protein or a variant thereof, or the Cas fusion protein are simultaneously added to the DNA binding reaction solution, and the solid substance is separated after coincubation. The solution in the method may be a solution used when the Cas protein or a variant thereof or Cas fusion protein binds to cell-free DNA, preferably the reaction time is from 30 minutes to 6 hours, and the reaction temperature is from room temperature (20-25°C) to 37°C. The elution conditions are the same as those in Method 1.

Examples

The examples are merely illustrative and are not intended to limit the present invention in any way.

Example 1: DNA binding activity of the Cas protein

The DNA binding activity of the Cas protein can be determined by using gel electrophoresis to compare the gel band positions of DNA binding with or without the Cas protein. The following reaction conditions were used:

reaction buffer (BA): 50 mM tris-hydroxymethylaminomethane hydrochloride (Tris-HCl, pH 7.4); 150 mM sodium chloride (NaCl)

reaction system (20 μl):

dCas protein: 2 μg

DNA: 0.25 μg

nuclease-free double-distilled water: the volume was made up to 20 μl

reaction process:

the above reaction system was added into a 200 μl PCR tube and mixed well, reacted at room temperature or 37°C for 15 minutes to 1 hour. After the reaction was completed, a non-denaturing gel was used and the electrophoresis was run in a BioRad electrophoresis tank at 80 V and 400 mA for 80 minutes. After removing the non-denaturing gel, the nucleic acid was stained with DuRed (Shanghai Yeasen Bio Technologies Co., Ltd., 10202ES76), and then a picture (see Figure 2) was taken by using a gel imager. The electrophoretic band of the DNA binding with Cas protein lagged behind that of the DNA binding without Cas protein significantly.

Example 2: Preparation of cell-free DNA

The cell-free DNA of the present invention can be DNA of any sequence. In this example, we synthesized and constructed 11 DNA sequences with different lengths, as shown in Table 1.

Table 1: Cell-free DNA sequence (5'-3')

| SEQ ID NO: | Length (bp) |
|---|---|
| 1 | 100 |
| 2 | 100 |
| 3 | 100 |
| 4 | 200 |
| 5 | 200 |
| 6 | 200 |
| 7 | 957 |
| 8 | 486 |
| 9 | 166 |
| 10 | 609 |
| 11 | 2655 |

The DNA sequences of SEQ ID NOs. 1-6 were synthesized by PCR directly with forward and reverse primers (Table 2), without using any template. The main purpose of using these six DNA sequences was to detect whether the protospacer-adjacent motif (PAM) region would affect the binding of the Cas protein to DNA. SEQ ID NOs. 1-3 were 100 bp, SEQ ID NOs. 4-6 were 200 bp, wherein there was no PAM region (NGG) corresponding to the Cas9 in SEQ ID NO. 1 and SEQ ID NO. 4, there was one PAM region on SEQ ID NO. 2 and SEQ ID NO. 5 respectively, and there were 3 PAM regions on SEQ ID NO. 3 and SEQ ID NO. 6 respectively. Detailed results will be discussed in Example 4.

The DNA sequences of SEQ ID NOs. 7-11 were made by PCR by designing the corresponding forward and reverse primers respectively, and using the 293T cell (Shanghai Institutes for Biological Sciences, GNHu17) genome as a template. Table 2: The upstream and downstream primers corresponding to different cell-free DNA sequences, and the annealing temperature of each PCR.

| | Upstream primer | Downstream primer | Annealing temperature (°C) |
|---|---|---|---|
| SEQ ID NO: 1 | SEQ ID NO: 12 | SEQ ID NO: 13 | 55 |
| SEQ ID NO: 2 | SEQ ID NO: 14 | SEQ ID NO: 15 | 55 |
| SEQ ID NO: 3 | SEQ ID NO: 16 | SEQ ID NO: 17 | 55 |
| SEQ ID NO: 4 | SEQ ID NO: 18 | SEQ ID NO: 19 | 58 |
| SEQ ID NO: 5 | SEQ ID NO: 20 | SEQ ID NO: 21 | 58 |
| SEQ ID NO: 6 | SEQ ID NO: 22 | SEQ ID NO: 23 | 58 |
| SEQ ID NO: 7 | SEQ ID NO: 24 | SEQ ID NO: 25 | 68 |
| SEQ ID NO: 8 | SEQ ID NO: 26 | SEQ ID NO: 27 | 60 |
| SEQ ID NO: 9 | SEQ ID NO: 28 | SEQ ID NO: 29 | 60 |
| SEQ ID NO: 10 | SEQ ID NO: 30 | SEQ ID NO: 31 | 59 |
| SEQ ID NO: 11 | SEQ ID NO: 32 | SEQ ID NO: 33 | 66 |

The PCR of each DNA sequence of SEQ ID NOs. 1-6 was performed by using two primers of upstream primer and downstream primer as described above and the Q5 hot-start ultra-fidelity 2X Master Mix kit (New England Biolabs, M0494S). The final concentration of each primer was 0.5 μM, 10 μl of Q5 2X Master Mix was added, and then water was added to a total volume of 50 μl, and then PCR was performed according to the manufacturer's guidelines. On the basis of the above reaction system, for the DNA sequences of SEQ ID NOs. 7-11, an additional 1 μl of cell genome extract was added, and the final volume was made up to 50 μl.

Example 3: Construction and purification of the dCas9 protein coupled with FLAG

1 Protein expression

1.1 Construction of expression vector: pSMART-his-S.P.dCas9-FLAG. The following was a schematic diagram of the vector loop, and the his-S P dCas9-FLAG gene fragment (SEQ ID No: 34) was integrated into the pSMART vector by EcoRI and BamHI restriction endonucleases (see Figure 1); if other linker sequences were used, then FLAG (FLAG sequence: GATTACAAGGATGACGATGACAAG) in the vector was replaced with the corresponding sequence, such as AVI-Tag, and other steps for expressing and purifying a protein were the same:
1.2 Selection of expression strains: Rosetta2 (NOVAGEN, 17400)
1.3 Culture conditions: Shaking culture

Instruments and consumables: shaker, LB medium (Biotechnology Co., Ltd, A507002)
Steps:
a plate (Rosetta/BL21) was taken out from the refrigerator at 4°C to be activate overnight at 37°C; transfer: 500ml of LB was inoculated with 4 ml of bacterial solution and incubated at 37°C for 4 hours, OD600 = 0.8, and taken out at room temperature. The shaker temperature was cooled to 18°C, 500 ul of IPTG (isopropyl-β-D-thiogalactopyranoside, Tiangen RT108-01) was added, and induced overnight with an induction time of 16 hours.

2 Protein purification

2.1 Cell harvest and lysis

Instruments and consumables: centrifuge, homogenizer, high-speed centrifuge, 0.45 μm filter membrane (Merck Millipore, SLHV033) and lysate
Steps: The cells were collected by centrifugation at 5000 rpm for 30 minutes, added with lysate to resuspend, and crushed by a homogenizer. The supernatant and precipitate were separated by high speed centrifugation. The supernatant was passed through a 0.45μm filter membrane and was ready for the next step of chromatographic purification.

2.2 Chromatography column purification process:
Instruments: AKTA-purify, chromatography column, chromatography solution

2.2.1 Histone affinity chromatography
Steps: A 20 ml histone affinity chromatography column was directly packed with a purchased medium (GE Life Sciences, 17-5318-02), and equilibrated with a 0.5% solution B (20 mM Tris-Hcl, PH = 8.0; 250 mM NaCl; 1 M imidazole). A purified protein solution was obtained according to the AKTA instrument instructions.
2.2.2 Cation exchange chromatography
Steps: A 20 ml cation exchange chromatography column (GE Life Sciences, 17-0407-01) was used, and equilibrated with a 0.5% solution B (20 mM HEPES-KOH PH = 7.5; 1 M KCL). A purified protein solution was obtained according to the AKTA instrument instructions.

3 Concentration and liquid change

Instruments and consumables: 30 kD concentration tube (Merck Millipore, UFC903096), low temperature high speed centrifuge
Steps: The protein solution was added to a concentration tube, and centrifuged at 4°C, 5000 rpm for 40 minutes; the concentration tube was taken out, the penetrating solution was removed, and 15 ml of protein preservation solution (20 mM Hepes, PH = 7.5; 150 mM KCl; 1% sucrose; 30% glycerol; 1 mM dithiothreitol (DTT)) was added, and the mixture was centrifuged at 4°C for 40 minutes; and the above steps were repeated 3 times to obtain the final his-S.P.dCas9-FLAG protein solution, which would be stored at -80°C after sub-packing.

Example 4: binding of cell-free DNA to the Cas protein

Under normal circumstances, the CRISPR/Cas system recognizes and cleaves specific DNA sequences based on the guidance of the PAM region (sequence: NGG, N may be A/T/C/G) and RNA. In order to verify whether the direct binding of the Cas protein and a variant thereof to a nucleic acid also depended on the PAM region, we prepared DNA sequences of SEQ ID NOs. 1-6. SEQ ID NOs.1-3 were 100 bp, SEQ ID NOs. 4-6 were 200 bp, of which there was no PAM region (NGG) corresponding to the Cas9 in SEQ ID NO. 1 and SEQ ID NO. 4, there was one PAM region on SEQ ID NO. 2 and SEQ ID NO. 5 respectively, and there was 3 PAM regions on SEQ ID NO. 3 and SEQ ID NO. 6 respectively. The dCas9 protein used in the reaction was the same as that in Examples 5, 6 and 7, which was the purified FLAG-dCas9 protein produced in Example 3. The reaction conditions were as followed:

reaction buffer (BA): 50 mM tris-hydroxymethylaminomethane hydrochloride (Tris-HCl, pH 7.4); 150 mM sodium chloride (NaCl)
reaction system:

total volume: 20 μl
dCas9 protein: 2 μg
DNA: 0.25 μg
Nuclease-free double distilled water: the volume was made up to 20 μl

Reaction process and result:
the above reaction system was added into a 200 μl PCR tube and mixed well, reacted at room temperature or 37°C for 15 minutes to 1 hour. After the reaction was completed, a non-denaturing gel was run in a BioRad electrophoresis tank at 80V and 400mA for 80 minutes. After removing the non-denaturing gel, the nucleic acid was stained with DuRed (Shanghai Yeasen Bio Technologies Co., Ltd., 10202ES76), and then a picture (Figure 2) was taken by using a gel imager. Figure 2 showed the binding electrophoresis band of cell-free DNA (SEQ ID NO. 7) of about 500 bp in length to a dCas protein. Lane 1 was a band of cell-free DNA itself and was a negative control. Lane 2 showed the situation after the dCas9 and cell-free DNA (SEQ ID NO. 7) were bound. In the negative control, the band of cell-free DNA was clear and complete, and the electrophoresis was successful. However, due to the binding of cell-free DNA to dCas9 in the second lane, DNA could not be electrophoresed normally, and almost all DNA was left in the lane well, therefore there was no obvious band at the corresponding position. In order to calculate the binding proportion (E1) of dCas9 to DNA, we used the following formula:

$$E1 = \frac{\text{Band brightness of lane 1} - \text{Band brightness at the corresponding position of lane 2}}{\text{Band brightness of lane 1}}$$

The formula used the band brightness of lane 1 as the benchmark for the total DNA amount, and the band brightness at the corresponding position of lane 2 as the amount of DNA not bound to the dCas9. So after the molecular calculation, it was the amount of DNA bound to the dCas9 protein, which was then divided by the total DNA amount to obtain the binding efficiency of the dCas9 to DNA, and the calculated binding efficiency of the dCas9 protein to DNA was 97.4%. Using the same experiment and calculation method, Table 3 summarized the dCas9-DNA binding proportions corresponding to different cell-free DNA lengths. The results showed that the dCas9 could efficiently bind to the above 11 types of DNA without difference, showing good potential for isolating nucleic acid from a solution.

Table 3: dCas9-DNA binding proportion corresponding to each DNA

| | SEQ ID NO. | | | | | | | | | | |
|---|---|---|---|---|---|---|---|---|---|---|---|
| | **1** | **2** | **3** | **4** | **5** | **6** | **7** | **8** | **9** | **10** | **11** |
| **DCAS9-DNA Binding proportion (E1)** | 97.1% | 96.9% | 96.1% | 96.9% | 97.0% | 96.8% | 97.1% | 97.4% | 98.4% | 97.2% | 96.4% |

Example 5: After the dCas9 protein coupled with FLAG was bound to cell-free DNA in a solution, it was bound to solid beads and isolated

reaction system:
total volume: 100 μl
dCas9 protein: 10 μg
DNA (SEQ ID NO. 7, 8, 9, 11, representing the length of different cell-free DNA): 0.25 μg of each DNA
Nuclease-free double distilled water: the volume was made up to 100 μl
the above reaction system was added into a 1.5 ml PCR tube and mixed well, reacted at room temperature or 37°C for 15 minutes to 1 hour.

15 μl of anti-FLAG affinity gel beads (Bimake, B23101) was added into a 1.5 ml centrifuge tube, 150 μl of the buffer solution (BA) in Example 4 was added, and the mixture was mixed well and centrifuged at 5000 RPM for 30 seconds. The supernatant was discarded, and the above step was repeated once. After the supernatant was discarded, the above 100 μl reaction system was added to the washed affinity gel beads, inverted and mixed well at room temperature, and reacted for 1 hour.

After the reaction was completed, the mixture was centrifuged at 5000 RPM for 30 seconds, the supernatant was discarded. 150 μl of the buffer solution (BA) in Example 4 was added, mixed well and centrifuged at 5000 RPM for 30 seconds. The supernatant was discarded, and the above step was repeated once. After the supernatant was discarded, 30 μl of proteinase K (Tiangen Biotech Co. Ltd, RT403) was added to the gel beads, and 20 μl of BA was added. After the mixture was mixed well, it was heated in a 55°C water bath for 30 minutes, and centrifuged at 5000 RPM for 30 seconds, and the supernatant was taken to obtain the isolated cell-free DNA. After gel electrophoresis and comparison with the positive control (0.25 μg of DNA was dissolved in 30 μl of proteinase K and 20 μl of BA solution), the DNA isolation efficiency E2 was calculated according to the following formula and summarized in Table 4:

$$E2 = \frac{\text{Brightness of DNA capture lane}}{\text{Brightness of positive control lane}}$$

Table 4: dCas9-DNA isolation efficiency corresponding to each DNA

| | **SEQ ID NO.** | | | |
|---|---|---|---|---|
| | **7** | **8** | **9** | **11** |
| **DNA isolation efficiency (E2)** | 79.5% | 77.2% | 77.6% | 70.9% |

Example 6: After the dCas9 protein coupled with FLAG was bound to the solid beads, cell-free DNA was bound and isolated from the solution

The first step: 15 μl of anti-FLAG affinity gel beads (GenScript, L00439-1) was added into a 1.5 ml centrifuge tube, 150 μl of the buffer solution (BA) in Example 4 was added, and the mixture was mixed well and centrifuged at 5000 RPM for 30 seconds. The supernatant was discarded, and the above step was repeated once. After the supernatant was discarded, 500 μl of BA was added to the gel beads and mixed well. Then 1 μl of 10 μg/μl dCas9 protein solution was added to the mixture, inverted and mixed well at room temperature, and reacted for 1 hour.

The second step: After the reaction was completed, the mixture was centrifuged at 5000 RPM for 30 seconds, the supernatant was discarded. 150 μl of the buffer solution (BA) in example 4 was added, mixed well and centrifuged at 5000 RPM for 30 seconds. The supernatant was discarded, and the above step was repeated once. The washed gel beads were added to 4 tubes of 1 ml solution containing cell-free DNA with different lengths (SEQ ID NOs. 7, 8, 9 and 11 were used for cell-free DNA, representing the lengths of different cell-free DNA were 957, 486, 166 and 2655 bp, respectively) respectively, the mixture was inverted, mixed well and reacted at room temperature or 37°C for 15 minutes to 1 hour.

The third step: After the reaction was completed, the mixture was centrifuged at 5000 RPM for 30 seconds, the supernatant was discarded. 150 μl of the buffer solution (BA) in Example 4 was added, mixed well and centrifuged at 5000 RPM for 30 seconds. The supernatant was discarded, and the above step was repeated once. After the supernatant was discarded, 30 μl of proteinase K (Tiangen Biotech Co. Ltd, RT403) was added to the gel beads, and 20 μl

of BA was added. After the mixture was mixed well, it was heated in a 55°C water bath for 30 minutes, and centrifuged at 5000 RPM for 30 seconds, and the supernatant was taken to obtain the isolated cell-free DNA.

In order to compare with QIAGEN's existing kits, cell-free DNA in a solution was extracted from the 1 ml of cell-free DNA solution in the second step by using the QIAamp® Circulating Nucleic Acid kit (QIAGEN Inc.) according to the steps in the instruction manual.

Positive control: The same amount of DNA (SEQ ID NOs. 7, 8, 9 and 11 were used for cell-free DNA, representing the lengths of different cell-free DNA were 957, 486, 166 and 2655 bp, respectively) was dissolved in 30 μl of proteinase K (Tiangen Biotech Co. Ltd, RT403), and 20 μl of BA was added.

The Agilent 2100 High Sensitivity DNA Analysis Kit (Cat. 5067-4626) was used to perform DNA quantitative analysis on DNA captured by the present invention, DNA captured by the Qiagen kit and the positive control. The results of amount and percentage of captured DNA were summarized in Table 4. The extraction efficiency was the ratio of the amount of DNA captured by the present invention or Qiagen kit in Table 4 to the amount of the positive control DNA (i.e., the percentage of captured DNA). The cell-free DNA extraction efficiency of the present invention was close to 80%, and the extraction efficiency of the QIAamp® Circulating Nucleic Acid kit was less than 50%, therefore the present invention increased the cell-free DNA extraction efficiency by about 60%.

Table 4: Comparison of the extraction efficiencies of cell-free DNA with various lengths by using the present invention and QIAamp® Circulating Nucleic Acid kit

| DNA size [BP] | The amount of DNA captured by the present invention [pg/μl] | The amount of DNA captured by the Qiagen kit [pg/μl] | DNA amount of the positive control [pg/μl] | The extraction efficiency of the present invention | The extraction efficiency of the Qiagen kit |
|---|---|---|---|---|---|
| **166** | 1,025.24 | 631.79 | 1,305.28 | 78.55% | 48.40% |
| **486** | 1,012.37 | 549.34 | 1,261.83 | 80.23% | 38.22% |
| **957** | 818.39 | 308.745 | 1077.58 | 75.95% | 28.65% |
| **2655** | 711.04 | 436.435 | 1179.08 | 60.30% | 28.65% |

Example 7: After the Cpfl (Cas12a) protein with His-Tag was bound to the solid beads, cell-free DNA was bound and isolated from the solution

The first step: The Cpfl (Cas12a) protein with His-Tag (IDT, Alt-R® As Cas12a (Cpfl) V3, Cat. No. 1081068) was linked to the His-Tag isolation magnetic beads (Dynabeads™ His-Tag Isolation and Pulldown, Cat. No. 10103D). 300 μl of Dynabeads were added into a 1.5 ml centrifuge tube, and 1 ml of pH = 7.4 PBS was added and mixed well. The centrifuge tube was placed in the corresponding sample hole of a magnetic separator (Beaver Company, Cat. No. 60201), and left to stand for two minutes, so that the magnetic beads were adsorbed and gathered on the wall of the tube, and the solution was restored to clarification. The supernatant was carefully aspirated, and the above steps were repeated twice. 40 μl of the Cpfl protein was mixed well with the Dynabeads, 1 ml of pH = 7.4 PBS was added, and the mixture was put on a mixer at room temperature to rotate for 20 minutes to 2 hours. The centrifuge tube was placed in the corresponding sample hole of a magnetic separator, and left to stand for two minutes. The supernatant was discarded, 500 μl of the buffer solution (BA) in Example 4 was added and mixed well. The centrifuge tube was placed in the corresponding sample hole of a magnetic separator, and left to stand for two minutes. The supernatant was discarded, and the above step was repeated once. 10 to 100 μl of the washed Dynabeads were added to 1 ml of a solution containing DNA of SEQ ID NO. 9, the mixture was inverted, mixed well and reacted at room temperature or 37°C for 15 minutes to 6 hours.

The second step: After the reaction was completed, the centrifuge tube containing the reaction solution and Dynabeads was placed in the corresponding sample hole of a magnetic separator, and left to stand for two minutes. The supernatant was discarded, and 150 μl of the buffer solution (BA) in example 4 was added. The centrifuge tube was placed in the corresponding sample hole of a magnetic separator, and left to stand for two minutes. The supernatant was discarded, and the above step was repeated once. After the supernatant was discarded, 30 μl of proteinase K (Tiangen Biotech Co. Ltd, RT403) was added to the Dynabeads, and 20 μl of BA was added. After the mixture was mixed well, it was heated in a 55°C water bath for 30 minutes. The centrifuge tube was placed in the corresponding sample hole of a magnetic separator, and left to stand for two minutes, and the centrifuge tube was placed in the sample hole corresponding to the magnetic separator, left to stand for two minutes, and the supernatant was taken to obtain the isolated cell-free DNA.

In order to compare with QIAGEN's existing kits, cell-free DNA in plasma was extracted from the 1 ml of cell-free DNA solution in the third step by using the QIAamp® Circulating Nucleic Acid kit (QIAGEN Inc.) according to the steps in the instruction manual.

Positive control: The same amount of DNA (SEQ ID NO. 9 was used for cell-free DNA, representing the length of cell-free DNA was 166 bp) was dissolved in 30 μl of proteinase K (Tiangen Biotech Co. Ltd, RT403), and 20 μl of BA was added.

The Agilent 2100 High Sensitivity DNA Analysis Kit (Cat. 5067-4626) was used to perform DNA quantitative analysis on the DNA captured by the present invention, the DNA captured by the Qiagen kit and the positive control. The results of amount and percentage of the captured DNA were summarized in Table 5. The extraction efficiency was the ratio of the amount of the DNA captured by the present invention or Qiagen kit in Table 5 to the amount of the positive control DNA (i.e., the percentage of the captured DNA). The cell-free DNA extraction efficiency of the present invention was close to 80%, and the extraction efficiency of the QIAamp® Circulating Nucleic Acid kit was only 51%, therefore the present invention increased the cell-free DNA extraction efficiency from plasma by about 54.7%.

Table 5: Comparison of the extraction efficiencies of cell-free DNA with 160 bp by using the present invention and QIAamp® Circulating Nucleic Acid kit

| DNA size [BP] | The amount of DNA captured by the present invention [pg/μl] | The amount of DNA captured by the QIAGEN KIT [pg/μl] | DNA amount of the . positive control [pg/μl] | extraction efficiency of the present invention | The extraction efficiency of the QIAGEN KIT |
|---|---|---|---|---|---|
| 166 | 1,018.62 | 658.34 | 1,293.72 | 78.74% | 50.89% |

Example 8: After the dCas9 protein coupled with AVI-Tag was bound to the solid beads, exogenous cell-free DNA was bound and isolated from plasma

The first step: For purification of the his-S.P.dCas9-AVI protein, example 3 was referred, and the FLAG sequence was replaced with the AVI-Tag sequence (DNA sequence: ggcctgaacgatatttttgaagcgcagaaaattgaatggcatgaa).

The second step: The AVI-Tag-dCas9 protein was biotinylated by using the BirA-500 kit (Cat. BirA500) from AViDITY according to the instructions of the kit. Corresponding reactants were added to a 1.5 ml centrifuge tube to react, and the reaction system was as follows:

| | |
|---|---|
| 10 × BiomixA | 8 μl |
| 10 × BiomixB | 8 μl |
| AVI-Tag-dCas9 protein | 50 μl |
| BirA enzyme | 0.8 μl |
| Ultra-pure water The total volume was made up to | 80 μl |

And then the reaction solution was reacted at room temperature for 1 hour or overnight.

The third step: The biotinylated protein was linked to Streptavidin gel beads. 400 μl of the GenScript Streptavidin gel beads (Cat. No. L00353) was placed in a 1.5 ml centrifuge tube, 1 ml of pH = 7.4 PBS was added, and the mixture was centrifuged at 5000g for 30 seconds at room temperature. After the centrifuge tube was taken out, the supernatant was carefully aspirated, and the above step was repeated twice. 80 μl of the biotinylated protein and the Streptavidin gel beads were mixed well, 1 ml of pH = 7.4 PBS was added, and the mixture was put on a mixer at room temperature to rotate for 20 minutes to 2 hours, and centrifuged at 5000 g at room temperature for 30 seconds. The supernatant was discarded. 500 μl of the buffer solution (BA) in example 4 was added, mixed well and centrifuged at 5000 RPM for 30 seconds. The supernatant was discarded, and the above step was repeated once. 10 to 100 μl of the washed gel beads were added to 1 ml of plasma containing DNA of SEQ ID NO. 9, the mixture was inverted, mixed well and reacted at room temperature or 37°C for 15 minutes to 6 hours.

The fourth step: After the reaction was completed, the mixture was centrifuged at 5000 RPM for 30 seconds, the supernatant was discarded. 150 μl of the buffer solution (BA) in example 4 was added, mixed well and centrifuged at 5000 RPM for 30 seconds. The supernatant was discarded, and the above step was repeated once. After the supernatant was discarded, 30 μl of proteinase K (Tiangen Biotech Co. Ltd, RT403) was added to the gel beads, and 20 μl of BA was added. After the mixture was mixed well, it was heated in a 55°C water bath for 30 minutes, and centrifuged at 5000 RPM for 30 seconds, and the supernatant was taken to obtain the isolated cell-free DNA.

In order to compare with QIAGEN's existing kits, cell-free DNA in plasma was extracted from the 1 ml of cell-free DNA solution in the third step by using the QIAamp® Circulating Nucleic Acid kit (QIAGEN Inc.) according to the steps in the instruction manual.

Positive control: The same amount of DNA (SEQ ID NO. 9 was used for cell-free DNA, representing the length of cell-free DNA was 166 bp) was dissolved in 30 μl of proteinase K (Tiangen Biotech Co. Ltd, RT403), and 20 μl of BA was added.

The Agilent 2100 High Sensitivity DNA Analysis Kit (Cat. 5067-4626) was used to perform DNA quantitative analysis on the DNA captured by the present invention, the DNA captured by the Qiagen kit and the positive control. The results of amount and percentage of the captured DNA were summarized in Table 6. The extraction efficiency was the ratio of the amount of the DNA captured by the present invention or Qiagen kit in Table 6 to the amount of the positive control DNA (i.e., the percentage of the captured DNA). The cell-free DNA extraction efficiency of the present invention was close to 78%, and the extraction efficiency of the QIAamp® Circulating Nucleic Acid kit was only 51%, therefore the present invention increased the cell-free DNA extraction efficiency from plasma by about 53%.

Table 6: Comparison of the extraction efficiencies of cell-free DNA with 160 bp by using the present invention and QIAamp® Circulating Nucleic Acid kit

| DNA size [BP] | The amount of DNA captured by the present invention [pg/μl] | The amount of DNA captured by the QIAGEN KIT [pg/μl] | DNA amount of the positive control [pg/μl] | The extraction efficiency of the present invention | The extraction efficiency of the QIAGEN KIT |
|---|---|---|---|---|---|
| 166 | 1,007.05 | 674.35 | 1,305.45 | 77.14% | 51.66% |

Example 9: After the Cas9 protein with His-Tag was bound to the solid beads, exogenous cell-free DNA was bound and isolated from plasma

The first step: The Cas9 protein with His-Tag (IDT, Alt-R® Sp Cas9 Nuclease V3, Cat. No. 1081058) was linked to the His-Tag isolation magnetic beads (Dynabeads ™ His-Tag Isolation and Pulldown, Cat. No. 10103D). 300 μl of Dynabeads were added into a 1.5 ml centrifuge tube, and 1 ml of pH = 7.4 PBS was added and mixed well. The centrifuge tube was placed in the corresponding sample hole of a magnetic separator (Beaver Company, Cat. No. 60201), and left to stand for two minutes, so that the magnetic beads were adsorbed and gathered on the wall of the tube, and the solution was restored to clarification. The supernatant was carefully aspirated, and the above steps were repeated twice. 40 μl of the Cas9 protein was mixed well with the Dynabeads, 1 ml of pH = 7.4 PBS was added, and the mixture was put on a mixer at room temperature to rotate for 20 minutes to 2 hours. The centrifuge tube was placed in the corresponding sample hole of a magnetic separator, and left to stand for two minutes. The supernatant was discarded, 500 μl of the buffer solution (BA) in example 4 was added and mixed well. The centrifuge tube was placed in the corresponding sample hole of a magnetic separator, and left to stand for two minutes. The supernatant was discarded, and the above step was repeated once. 10 to 100 μl of the washed Dynabeads were added to 1 ml of plasma containing DNA of SEQ ID NO. 9, the mixture was inverted, mixed well and reacted at room temperature or 37°C for 15 minutes to 6 hours.

The second step: After the reaction was completed, the centrifuge tube containing plasma and Dynabeads was placed in the corresponding sample hole of a magnetic separator, and left to stand for two minutes. The supernatant was discarded, and 150 μl of the buffer solution (BA) in example 4 was added. The centrifuge tube was placed in the corresponding sample hole of a magnetic separator, and left to stand for two minutes. The supernatant was discarded, and the above step was repeated once. After the supernatant was discarded, 30 μl of proteinase K (Tiangen Biotech Co. Ltd, RT403) was added to the Dynabeads, and 20 μl of BA was added. After the mixture was mixed well, it was heated in a 55°C water bath for 30 minutes. The centrifuge tube was placed in the corresponding sample hole of a magnetic separator, and left to stand for two minutes, and the centrifuge tube was placed in the sample hole corresponding to the magnetic separator, left to stand for two minutes, and the supernatant was taken to obtain the isolated cell-free DNA.

In order to compare with QIAGEN's existing kits, cell-free DNA in plasma was extracted from the 1 ml of cell-free DNA solution in the third step by using the QIAamp® Circulating Nucleic Acid kit (QIAGEN Inc.) according to the steps in the instruction manual.

Positive control: The same amount of DNA (SEQ ID NO. 9 was used for cell-free DNA, representing the length of cell-free DNA was 166 bp) was dissolved in 30 μl of proteinase K (Tiangen Biotech Co. Ltd, RT403), and 20 μl of BA was added.

The Agilent 2100 High Sensitivity DNA Analysis Kit (Cat. 5067-4626) was used to perform DNA quantitative analysis on the DNA captured by the present invention, the DNA captured by the Qiagen kit and the positive control. The results of amount and percentage of the captured DNA were summarized in Table 7. The extraction efficiency was the ratio of the amount of the DNA captured by the present invention or Qiagen kit in Table 7 to the amount of the positive control DNA (i.e., the percentage of the captured DNA). The cell-free DNA extraction efficiency of the present invention exceeded 85%, and the extraction efficiency of the QIAamp® Circulating Nucleic Acid kit was only 55%, therefore the present invention increased the cell-free DNA extraction efficiency from plasma by about 54.5%.

Table 7: Comparison of the extraction efficiencies of cell-free DNA with 160 bp by using the present invention and QIAamp® Circulating Nucleic Acid kit

| DNA size [BP] | The amount of DNA captured by the present invention [pg/µl] | The amount of DNA captured by the QIAGEN KIT [pg/µl] | DNA amount of the , positive control [pg/µl] | extraction efficiency of the present invention | The extraction efficiency of the QIAGEN KIT |
|---|---|---|---|---|---|
| 170 | 1,160.66 | 754.54 | 1,363.28 | 85.14% | 55.35% |

Example 10: After the dCas9 protein coupled with AVI-Tag was bound to the solid beads, endogenous cell-free DNA was bound and isolated from plasma

The first step and the second step were the same as the first step and the second step in example 8, and a biotinylated dCas9 protein was obtained.

The third step: The biotinylated protein was linked to Streptavidin gel beads. 400 µl of the GenScript Streptavidin gel beads (Cat. No. L00353) was placed in a 1.5 ml centrifuge tube, 1 ml of pH = 7.4 PBS was added, and the mixture was centrifuged at 5000g for 30 seconds at room temperature. After the centrifuge tube was taken out, the supernatant was carefully aspirated, and the above step was repeated twice. 80 µl of the biotinylated protein and the Streptavidin gel beads were mixed well, 1 ml of pH = 7.4 PBS was added, and the mixture was put on a mixer at room temperature to rotate for 20 minutes to 2 hours, and centrifuged at 5000 g at room temperature for 30 seconds. The supernatant was discarded. 500 µl of the buffer solution (BA) in example 4 was added, mixed well and centrifuged at 5000 RPM for 30 seconds. The supernatant was discarded, and the above step was repeated once. 10 to 100 µl of the washed gel beads were added to 1 ml of plasma, the mixture was inverted, mixed well and reacted at room temperature or 37°C for 15 minutes to 6 hours.

The fourth step: After the reaction was completed, the mixture was centrifuged at 5000 RPM for 30 seconds, the supernatant was discarded. 150 µl of the buffer solution (BA) in example 4 was added, mixed well and centrifuged at 5000 RPM for 30 seconds. The supernatant was discarded, and the above step was repeated once. After the supernatant was discarded, 30 µl of proteinase K (Tiangen Biotech Co. Ltd, RT403) was added to the gel beads, and 20 µl of BA was added. After the mixture was mixed well, it was heated in a 55°C water bath for 30 minutes, and centrifuged at 5000 RPM for 30 seconds, and the supernatant was taken to obtain the isolated cell-free DNA.

In order to compare with QIAGEN's existing kits, cell-free DNA in plasma was extracted from the 1 ml of cell-free DNA solution in the third step by using the QIAamp® Circulating Nucleic Acid kit (QIAGEN Inc.) according to the steps in the instruction manual.

The Agilent 2100 High Sensitivity DNA Analysis Kit (Cat. 5067-4626) was used to perform DNA quantitative analysis on DNA captured by the present invention and DNA captured by the Qiagen kit. The results of captured DNA amount were summarized in Table 8. The cell-free DNA extraction amount of the present invention was close to 28.49 pg/µl, and the extraction efficiency of the QIAamp® Circulating Nucleic Acid kit was only 24.68 pg/µl, therefore the present invention increased the endogenous cell-free DNA extraction efficiency from plasma by about 15.4%.

Table 8: Comparison of the extraction amounts of cell-free DNA in plasma by using the present invention and QIAamp® Circulating Nucleic Acid kit

| DNA size [BP] | The amount of DNA captured by the present invention [pg/µl] | The amount of DNA captured by the QIAGEN KIT [pg/µl] | The extraction efficiency of the present invention increased by |
|---|---|---|---|
| 177 | 28.49 | 24.68 | 15.4% |

A 1 st embodiment according to the present invention is defined in independent claim 1 The denendent claims define preferred embodiments of the invention

## Claims

1. A method for isolating DNA from a DNA solution by using a Cas protein in the absence of RNA mediation, comprising the steps of a) mixing the DNA solution with a Cas protein system; and c) isolating DNA from the complex formed by the binding of the Cas protein system with DNA.

2. The method of claim 1, comprising the steps of a) mixing the DNA solution with a Cas protein system; b) enriching the complex formed by the binding of the Cas protein system with DNA in step a); and c) isolating DNA from the enriched complex in step b).

3. The method of claim 1, wherein the Cas protein system is a Cas protein coupled with a plate-like, film-like or columnar solid support.

4. The method of claim 2, wherein the Cas protein system is a Cas protein coupled with a bead-shaped solid support.

5. The method of claim 2, wherein the Cas protein system is a Cas fusion protein formed by the Cas protein and at least one linker sequence.

6. The method of claim 5, wherein in step b), a solid support coupled with an affinity molecule is added to the solution, then the enrichment is performed by centrifugation or magnetism.

7. The method of claim 5, wherein a solid support coupled with an affinity molecule is added in step a), then the enrichment is performed, after incubation, in step b).

8. The method of any one of claims 1-7, wherein the DNA is double-stranded DNA.

9. The method of claim 3 or 4, wherein the Cas protein system is formed by coupling the Cas protein to a solid support through a chemical covalent bond.

10. The method of claim 5, wherein the Cas protein system is formed by combining the Cas fusion protein with the affinity molecule on the solid support.

11. The method of any one of claims 3-10, wherein the solid support is a solid substance capable of forming a chemical bond with an amino acid residue.

12. The method of claim 9, wherein the solid support is selected from at least one of solid substances made of gel materials, magnetic materials, cellulose materials, silica gel materials, glass materials and artificial high-molecular polymers.

13. The method of any one of claims 5-12, wherein the linker sequence and the affinity molecule are selected from one of the following combinations of ligand and receptor: enzyme and substrate, antigen and antibody, and biotin and avidin.

14. A kit for isolating DNA from a solution, in the absence of RNA mediation, comprising components a) a Cas protein system, and b) a DNA eluent, wherein component a) the Cas protein system is a solution system containing a Cas protein coupled with a solid support, or is a solution system containing a Cas fusion protein and a solid support coupled with an affinity molecule.

**Patentansprüche**

1. Verfahren zur Isolierung von DNA aus einer DNA-Lösung unter Verwendung eines Cas-Proteins in Abwesenheit von RNA-Vermittlung, umfassend die Schritte a) Mischen der DNA-Lösung mit einem Cas-Protein-System; und c) Isolieren von DNA aus dem Komplex, der durch die Bindung des Cas-Protein-Systems mit DNA gebildet wird.

2. Verfahren nach Anspruch 1, umfassend die Schritte a) Mischen der DNA-Lösung mit einem Cas-Proteinsystem; b) Anreichern des Komplexes, der durch die Bindung des Cas-Proteinsystems mit DNA in Schritt a) gebildet wird; und c) Isolieren von DNA aus dem angereicherten Komplex in Schritt b).

3. Verfahren nach Anspruch 1, wobei das Cas-Protein-System ein Cas-Protein ist, das mit einem plattenförmigen, filmartigen oder säulenförmigen festen Träger gekoppelt ist.

4. Verfahren nach Anspruch 2, wobei das Cas-Protein-System ein Cas-Protein ist, das mit einem kugelförmigen festen Träger gekoppelt ist.

5. Verfahren nach Anspruch 2, wobei das Cas-Protein-System ein Cas-Fusionsprotein ist, das durch das Cas-Protein und mindestens eine Linker-Sequenz gebildet wird.

6. Verfahren nach Anspruch 5, wobei in Schritt b) ein fester Träger, der mit einem Affinitätsmolekül gekoppelt ist, zu der Lösung gegeben wird und dann die Anreicherung durch Zentrifugation oder Magnetismus durchgeführt wird.

7. Verfahren nach Anspruch 5, wobei in Schritt a) ein fester Träger, der mit einem Affinitätsmolekül gekoppelt ist, zugegeben wird und dann die Anreicherung nach der Inkubation in Schritt b) durchgeführt wird.

8. Verfahren nach einem der Ansprüche 1 bis 7, wobei die DNA eine doppelsträngige DNA ist.

9. Verfahren nach Anspruch 3 oder 4, wobei das Cas-Protein-System durch Kopplung des Cas-Proteins an einen festen Träger durch eine chemisch kovalente Bindung gebildet wird.

10. Verfahren nach Anspruch 5, wobei das Cas-Protein-System durch Kombination des Cas-Fusionsproteins mit dem Affinitätsmolekül auf dem festen Träger gebildet wird.

11. Verfahren nach einem der Ansprüche 3 bis 10, wobei der feste Träger eine feste Substanz ist, die in der Lage ist, eine chemische Bindung mit einem Aminosäurerest zu bilden.

12. Verfahren nach Anspruch 9, wobei der feste Träger ausgewählt ist aus mindestens einer der festen Substanzen aus Gelmaterialien, magnetischen Materialien, Zellulosematerialien, Kieselgelmaterialien, Glasmaterialien und künstlichen hochmolekularen Polymeren.

13. Verfahren nach einem der Ansprüche 5 bis 12, wobei die Linkersequenz und das Affinitätsmolekül aus einer der folgenden Kombinationen von Ligand und Rezeptor ausgewählt sind: Enzym und Substrat, Antigen und Antikörper sowie Biotin und Avidin.

14. Kit zur Isolierung von DNA aus einer Lösung ohne RNA-Vermittlung, umfassend die Komponenten a) ein Cas-Protein-System und b) ein DNA-Eluent, wobei Komponente a) das Cas-Proteinsystem ein Lösungssystem ist, das ein Cas-Protein gekoppelt mit einem festen Träger enthält, oder ein Lösungssystem ist, das ein Cas-Fusionsprotein und einen festen Träger gekoppelt mit einem Affinitätsmolekül enthält.

## Revendications

1. Procédé pour isoler l'ADN d'une solution d'ADN en utilisant une protéine Cas en l'absence de médiation ARN, comprenant les étapes de a) mélanger la solution d'ADN avec un système de protéine Cas ; et c) isoler l'ADN du complexe formé par la liaison du système de protéine Cas avec l'ADN.

2. Procédé selon la revendication 1, comprenant les étapes de a) mélanger la solution d'ADN avec un système de protéine Cas ; b) enrichir le complexe formé par la liaison du système de protéine Cas avec l'ADN dans l'étape a) ; et c) isoler l'ADN du complexe enrichi dans l'étape b).

3. Procédé selon la revendication 1, dans lequel le système de protéine Cas est une protéine Cas couplée à un support solide en forme de plaque, de film ou de colonne.

4. Procédé selon la revendication 2, dans lequel le système de protéine Cas est une protéine Cas couplée à un support solide en forme de bille.

5. Procédé selon la revendication 2, dans lequel le système de protéine Cas est une protéine de fusion Cas formée par la protéine Cas et au moins une séquence de liaison.

6. Procédé selon la revendication 5, dans lequel à l'étape b), un support solide couplé à une molécule d'affinité est ajouté à la solution, puis l'enrichissement est réalisé par centrifugation ou magnétisme.

7. Procédé selon la revendication 5, dans lequel un support solide couplé à une molécule d'affinité est ajouté à l'étape a), puis l'enrichissement est réalisé, après incubation, à l'étape b).

8. Procédé selon l'une quelconque des revendications 1 à 7, dans lequel l'ADN est un ADN double brin.

9. Procédé selon la revendication 3 ou 4, dans lequel le système de protéine Cas est formé en couplant la protéine Cas à un support solide par une liaison covalente chimique.

10. Procédé selon la revendication 5, dans lequel le système de protéine Cas est formé en combinant la protéine de fusion Cas avec la molécule d'affinité sur le support solide.

11. Procédé selon l'une quelconque des revendications 3 à 10, dans lequel le support solide est une substance solide capable de former une liaison chimique avec un résidu d'acide aminé.

12. Procédé selon la revendication 9, dans lequel le support solide est sélectionné parmi au moins l'une des substances solides faites de matériaux de gel, de matériaux magnétiques, de matériaux de cellulose, de matériaux de gel de silice, de matériaux de verre et de polymères artificiels de haut poids moléculaire.

13. Procédé selon l'une quelconque des revendications 5 à 12, dans lequel la séquence de liaison et la molécule d'affinité sont sélectionnées parmi l'une des combinaisons suivantes de ligand et récepteur : enzyme et substrat, antigène et anticorps, et biotine et avidine.

14. Kit pour isoler l'ADN d'une solution, en l'absence de médiation ARN, comprenant les composants a) un système de protéine Cas, et b) un éluant d'ADN, dans lequel le composant a) le système de protéine Cas est un système de solution contenant une protéine Cas couplée à un support solide, ou est un système de solution contenant une protéine de fusion Cas et un support solide couplé à une molécule d'affinité.

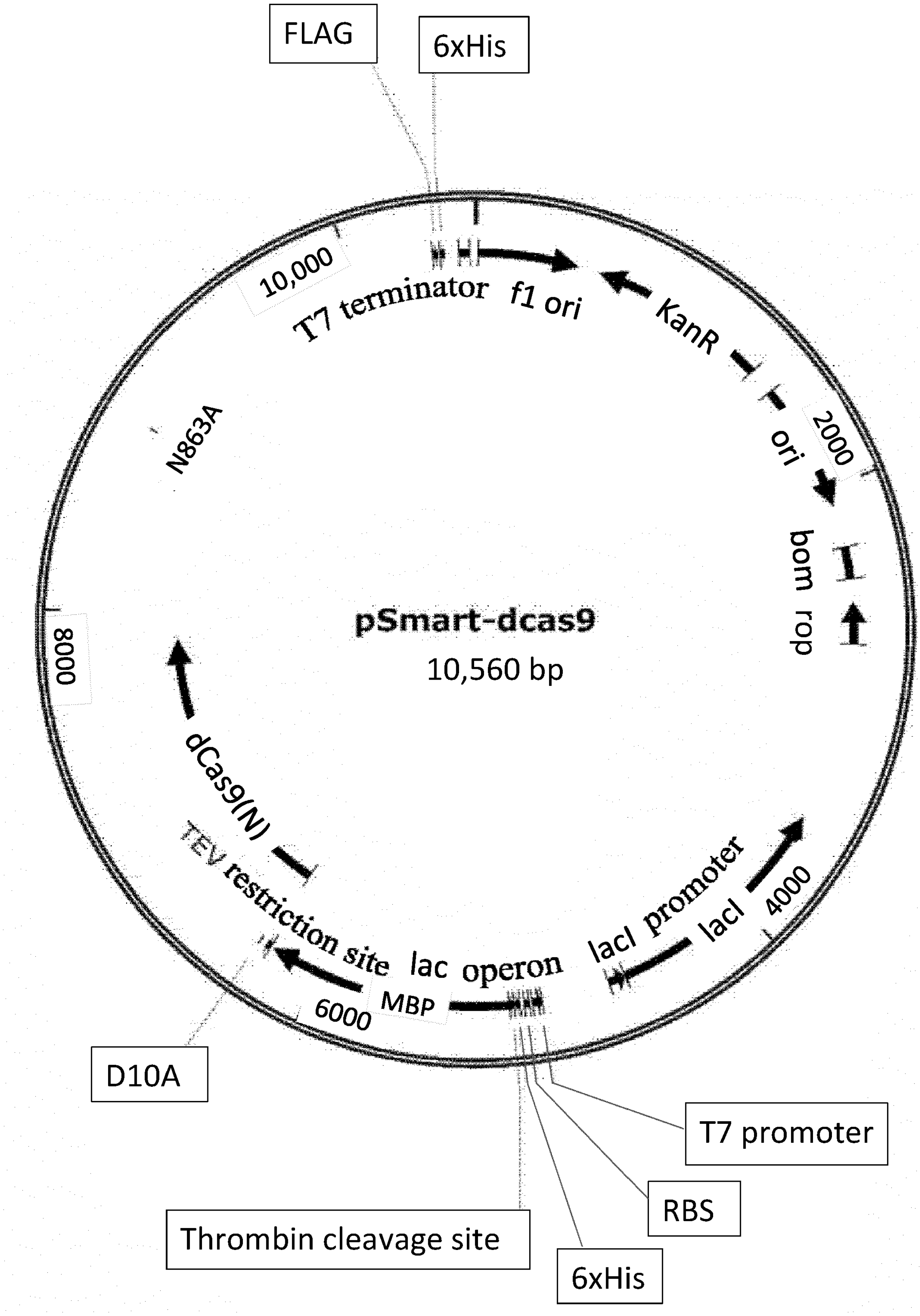
FLAG
6xHis
10,000
T7 terminator
f1 ori
KanR
ori
2000
bom
rop
N863A
pSmart-dcas9
10,560 bp
8000
dCas9(N)
TEV restriction site
lac operon
lacI promoter
lacI
4000
MBP
6000
D10A
T7 promoter
RBS
Thrombin cleavage site
6xHis

*Fig. 1*

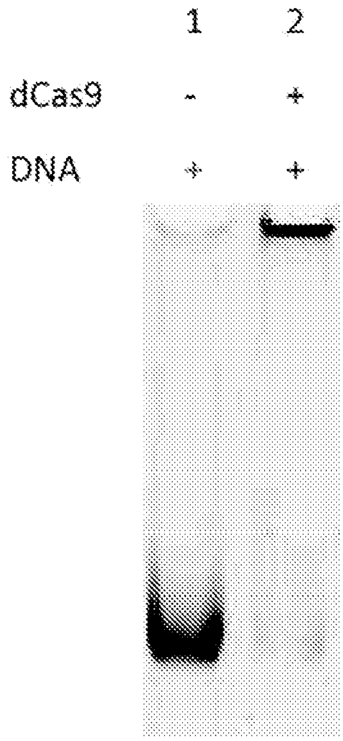


*Fig. 2*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016198571 A1
- WO 2015120445 A1
- US 2014356867 A1


### Non-patent literature cited in the description


- **MANDEL P.** *C R Hebd Seances Acad Sci (Paris),* 1948, vol. 142 (3), 241-3
- **XUE et al.** *Clinica Chimica Acta,* 2009, vol. 404, 100-104
- **CONG et al.** *Science,* 2013, vol. 339, 819-823
- **JINEK et al.** *Science,* 2012, vol. 337, 816-821
- In Situ Capture of Chromatin Interactions by Biotinylated dCas9. **LIU XIN et al.** CELL. ELSEVIER, 25 August 2017, vol. 170, 1028
- **ZACHARY WALDRIP et al.** A CRISPR-based approach for proteomic analysis of a single genomic locus. *EPIGENETICS,* 18 July 2014, vol. 9 (9), ISSN 1559-2294, 1207-1211
- Efficient isolation of specific genomic regions and identification of associated proteins by engineered DNA-binding molecule-mediated chromatin immunoprecipitation (enChIP) using CRISPR. **TOSHITSUGU FUJITA et al.** BIOCHEMICAL AND BIOPHYSICAL RESEARCH COMMUNICATIONS. ELSEVIER, 11 August 2013, vol. 439, 132-136
- **CHRISTOPHER A. V et al.** *Nature Medicine,* 2018, (24), 1216-1224
- **JOHNNY H. H et al.** *Nature,* 2018, vol. 556 (7699), 57-63
- **JANA M et al.** *Plant Biotechnology Journal,* 2017, vol. 15, 917-926
- **MA et al.** *ACS Synth. Biol.,* 2018, vol. 7 (4), 978-985
- **KOMOR et al.** *Sci. Adv.,* 2017, vol. 3, eaao4774
- **QI et al.** *Cell,* 2013, vol. 152, 1173-1183
- **HERMANSON et al.** Immobilized Affinity Ligand Techniques. Academic Press, 1992

SEQUENCE LISTING

<110> 苏州克睿基因生物科技有限公司

<120> **一种** Cas **蛋白体系分离** DNA**的方法**

<130> AJFS2017BJ692

<160> 34

<170> PatentIn version 3.5

<210> 1
<211> 100
<212> DNA
<213> synthetic sequence

<400> 1

**ctgcgtaaac gtcgctgtgc tagctctttt gcagtcacag cttttcgtgc atgagtacgt 60**

**attttgaaac tcaagatcgc attcatgcgt cttcacgtga 100**

<210> 2
<211> 100
<212> **DNA**
<213> synthetic sequence

<400> 2

**ctgcgtaaac gtcgctgtgc tagctctttt gcaggcacag cttttcgtgc atgagtacgt 60**

**attttgaaac tcaagatcgc attcatgcgt cttcacgtga 100**

<210> 3
<211> 100
<212> **DNA**
<213> synthetic sequence

<400> 3

**ctgcgtaaac gtcgcggtgc tagctctttt gcaggcacag cttttcgtgc atgagtatgg 60**

**attttgaaac tcaagatcgc attcatgcgt cttcacgtga 100**

<210> 4
<211> 200
<212> DNA
<213> synthetic sequence

<400> 4

```
ctgcttcgtg catgagtacg tattctcttt acgtgactgc gtaagtaaac gtcgctgtgc      60
tagtgcagtc acagttgaaa ctcaagatcg cattcaagct ctttactgct tacgtgactg     120
cgttgcagtc acagcttagt acgtattgcg tcttcagtgc atgttgaaac tcaagatcgc     180
attcatgcgt cttcacgtga                                                 200
```

<210> 5
<211> 200
<212> **DNA**
<213> synthetic sequence

<400> 5

```
ctgcttcgtg catgagtacg tattctcttt acgtgactgc gtaagtaaac gtcgctgtgc      60
tagtgcagtc acagttgaaa ctcaagatcg cattcaaggt ctttactgct tacgtgactg     120
cgttgcagtc acagcttagt acgtattgcg tcttcagtgc atgttgaaac tcaagatcgc     180
attcatgcgt cttcacgtga                                                 200
```

<210> 6
<211> 200
<212> **DNA**
<213> synthetic sequence

<400> 6

```
ctgcttcgtg catgcggacg tattctcttt acgtgactgc gtaagtaaac gtcgctgtgc      60
tagtgcagtc acagttgaaa ctcaagatcg cattcaaggt cgttactgct tacgtgactg     120
cgttgcagtc acagcttagt acgtattgcg tcttcagtgc atgttgaaac tcaaggtcgc     180
attcatgcgt cttcacgtga                                                 200
```

<210> 7
<211> 957
<212> **DNA**
<213> Homo sapiens

<400> 7

```
gccctcatga tattttaaaa cacagcatcc tcaaccttga ggcggaggtc ttcataacaa        60
agatactatc agttcccaaa ctcagagatc aggtgactcc gactcctcct ttatccaatg       120
tgctcctcat ggccactgtt gcctgggcct ctctgtcatg gggaatcccc agatgcaccc       180
aggaggggcc ctctcccact gcatctgtca cttcacagcc ctgcgtaaac gtccctgtgc       240
taggtctttt gcaggcacag cttttcctcc atgagtacgt attttgaaac tcaagatcgc       300
attcatgcgt cttcacctgg aaggggtcca tgtgcccctc cttctggcca ccatgcgaag       360
ccacactgac gtgcctctcc ctccctccag gaagcctacg tgatggccag cgtggacaac       420
ccccacgtgt gccgcctgct gggcatctgc ctcacctcca ccgtgcagct catcacgcag       480
ctcatgccct tcggctgcct cctggactat gtccgggaac acaaagacaa tattggctcc       540
cagtacctgc tcaactggtg tgtgcagatc gcaaaggtaa tcagggaagg gagatacggg       600

gaggggagat aaggagccag gatcctcaca tgcggtctgc gctcctggga tagcaagagt       660
ttgccatggg gatatgtgtg tgcgtgcatg cagcacacac acattccttt attttggatt       720
caatcaagtt gatcttcttg tgcacaaatc agtgcctgtc ccatctgcat gtggaaactc       780
tcatcaatca gctacctttg aagaattttc tctttattga gtgctcagtg tggtctgatg       840
tctctgttct tatttctctg gaattctttg tgaatactgt ggtgatttgt agtggagaag       900
gaatattgct tcccccattc aggacttgat aacaaggtaa gcaagccagg ccaaggc          957
```

<210> 8
<211> 486
<212> DNA
<213> Homo sapiens

<400> 8

ctctcccact gcatctgtca cttcacagcc ctgcgtaaac gtccctgtgc taggtctttt 60

gcaggcacag cttttcctcc atgagtacgt attttgaaac tcaagatcgc attcatgcgt 120

cttcacctgg aaggggtcca tgtgcccctc cttctggcca ccatgcgaag ccacactgac 180

gtgcctctcc ctccctccag gaagcctacg tgatggccag cgtggacaac cccacgtgt 240

gccgcctgct gggcatctgc ctcacctcca ccgtgcagct catcacgcag ctcatgccct 300

tcggctgcct cctggactat gtccgggaac acaaagacaa tattggctcc cagtacctgc 360

tcaactggtg tgtgcagatc gcaaaggtaa tcagggaagg gagatacggg gaggggagat 420

aaggagccag gatcctcaca tgcggtctgc gctcctggga tagcaagagt ttgccatggg 480

gatatg 486

<210> 9
<211> 166
<212> **DNA**
<213> Homo sapiens

<400> 9

ctccaggaag cctacgtgat ggccagcgtg gacaaccccc acgtgtgccg cctgctgggc 60

atctgcctca cctccaccgt gcagctcatc acgcagctca tgcccttcgg ctgcctcctg 120

gactatgtcc gggaacacaa agacaatatt ggctcccagt acctgc 166

<210> 10
<211> 609
<212> DNA
<213> Homo sapiens

<400> 10

tctccacaag gaggcatgga aaggctgtag ttgttcacct gcccaagaac taggaggtct 60

ggggtgggag agtcagcctg ctctggatgc tgaaagaatg tctgtttttc cttttagaaa 120

gttcctgtga tgtcaagctg gtcgagaaaa gctttgaaac aggtaagaca ggggtctagc 180

ctgggtttgc acaggattgc ggaagtgatg aacccgcaat aaccctgcct ggatgaggga 240

gtgggaagaa attagtagat gtgggaatga atgatgagga atggaaacag cggttcaaga 300

cctgcccaga gctgggtggg gtctctcctg aatccctctc accatctctg actttccatt 360

ctaagcactt tgaggatgag tttctagctt caatagacca aggactctct cctaggcctc 420

tgtattcctt tcaacagctc cactgtcaag agagccagag agagcttctg ggtggcccag 480

ctgtgaaatt tctgagtccc ttagggatag ccctaaacga accagatcat cctgaggaca 540

gccaagaggt tttgccttct ttcaagacaa gcaacagtac tcacataggc tgtgggcaat 600

ggtcctgtc 609

<210> 11
<211> 2655
<212> DNA
<213> Homo sapiens

<400> 11

cgttccagaa gcggcagaag cttacctccg agggtgccgc caagctcctg ctagacacct 60

tgtgagtgcg gcgggccggg gggcgcggga gccgttgcca cgcggacccc ctcggcagga 120

gtcgggctcg cagcccgcgt gcaggccttg ggcgcctttc agctctgagc cttccacgtt 180

acggagccga cgcggctccc ccgttagcac tggggttatt ccttaattct aagggcggcg 240

gggcggggcg gtgcttctag ggctgtgtgg cctgggaatg gaaaggggc ttcgtccgga 300

gcttgagggc gcgcgcagcc gtcttgggac acaaagagtc tttcccggcc ataagcatcg 360

tgctcgggga cgttgtctct tgctggcgcg caaagggtgt gggggccttt cttagtgaaa 420

agaacataaa tcccacggct ttctcttacc cgaatcccct tttttttgt gtttgcacac 480

gtgtttttga gggtgggtgg aagggaaagg tagaagcgtt gaaggaagca tgaatagttt 540

tgaactcact gggaagcaaa atcccgtcat tgttccaggc actgccaggg agggagcagg 600

gggcggaggt tattgatccg ggagagaagc gcgcgcgaag agatgctccg gcctcgcgcg 660

gcggcgggga tacctacgta gggaggcttt tatgtgctca gccagctaat gctccctgcc 720

gctcgccctc ctggctgctc cgcagtcttt tagatggccc gaggagcatc tcgagtgtcc 780

gcgatttggg aaagagcttt taactgggct gttaaagggc tgccttcttt tctccaaatg 840

cgatctggat tcctgcctcg ggaacagtt gtgctacaga gagatatttt aacgccctga 900

tattatattt cagtgtcaat tttacgaatt aaaaagaaca atcaaaacac taggggggaaa 960
aaaccgacaa gaataaccta gcaattgcat aacctgagaa ttacttcttt tcttgaacat 1020
gttcctttga gcttcttgcg gaagaaattc tcttaggatg agtcgtcccc gacagaagat 1080
acatatttgg ttttctgtgt gcttcacttt ttggtttttc atcttggact gcacactgaa 1140
tttttaaaaa acgttcatcc atcaaacatt tgagcaccta tagggtgacg gctgcctctg 1200
ggcagaagga atacgaagat ccttaggaat gtaaaactca tggtgtcttc aaggcataca 1260
gaaaaaaaga agaatgtaaa actcaaattt gagataaatt tttattttaa gacaaactga 1320
taaatggtca agaaaaagtt ttaaaatatt gacataaaaa agctgttttt ctcccactag 1380
attgccatga ctttgtactt ataaagtcta ctaaattata ttcaaaaagt gtgtataact 1440
gtaccatttt cgttaaaata ttgtgtagaa aaaagtttgg gggaatatat aacaaataat 1500
taactgtaga tccctctggg tgtaaagatt acaggaggct ttcactttga gcactctcaa 1560
atagtgtgaa gtttggagtt tttacaataa gcattatgtg tgcagaaaaa attaaaatac 1620
ataaacgtaa agaaacatga aaaaatgatc agtcttaatc atgcaaatta aaactgcagt 1680
gagctatttt tcctataatt attgcctgag actgtacagt gaaatgtttt ctcatttatt 1740
tatcgggtaa gggtgttcaa tccttttgga aagcgatttt aagacattta tcagatctta 1800
atgttcattt ctgagtttgg cttatatata agctttaaag ggttaaaatt gtatgtaggc 1860
tgggtgaggt ggctcatgcc tgtaatccca gcactttggg aggccgaggc gggtggatca 1920
cctgaggtgg ggagtttgag actgcctgac caacatggag aaaccccgtc tctactaaaa 1980
atacaaaatt agctgggtgt ggtggtgggc gcctgtaatc ccagctactc gggaggctga 2040
ggcaggagaa tcatttgaac cctggaggtg gaggttgcgg tgggctgaaa tcgcgtcact 2100
gcactccagc ctgggcaaca agaatgaaac tccctctcaa aaaaaaaaaa ttctatgtaa 2160
tgtgtattac aactatgtaa aacgcatgtg tatgaagaat actggaaaga aatagagcaa 2220
aaatataaga gcctttatta agtttctcaa aaggctcttc tggacctctg gacttgattg 2280
agtacacagt cttatttttc atagcagcct gtacctcctt tatcacaatc ataattaatt 2340
attatttgag taaattgtta aggtcggtaa gggtagcaat cgtgtctgct gtatcttgtt 2400
tagtgttctc tccttcgtgc ctagctcata aaaatattta gtatttgagg cccggagcag 2460
tggctcacgc ctgtaatccc agcactttgg gagtccccgt gggtggatca caaggtcagg 2520
agttcaagac cagcttggcc aagatggtga agccctatct ctactaaaaa tacaaaatta 2580
gcagggcgcg gtggcaggcg cctgtaatcc cagctacttg ggaggctaag gctggagaat 2640

tgcttcaacc caggt 2655

<210> 12
<211> 60
<212> DNA
<213> synthetic sequence

<400> 12
ctgcgtaaac gtcgctgtgc tagctctttt gcagtcacag cttttcgtgc atgagtacgt 60

<210> 13
<211> 62
<212> DNA
<213> synthetic sequence

<400> 13

agcttttcgt gcatgagtac gtattttgaa actcaagatc gcattcatgc gtcttcacgt 60

ga 62

<210> 14
<211> 72
<212> DNA
<213> synthetic sequence

<400> 14

ctgcgtaaac gtcgctgtgc tagctctttt gcaggcacag cttttcgtgc atgagtacgt 60

attttgaaac tc 72

<210> 15
<211> 55
<212> DNA
<213> synthetic sequence

<400> 15
cgtgcatgag tacgtatttt gaaactcaag atcgcattca tgcgtcttca cgtga 55

<210> 16
<211> 63
<212> DNA
<213> synthetic sequence

<400> 16

ctgcgtaaac gtcgcggtgc tagctctttt gcaggcacag cttttcgtgc atgagtatgg 60

att 63

<210> 17
<211> 61
<212> **DNA**

<213> synthetic sequence

<400> 17

**gcttttcgtg catgagtatg gattttgaaa ctcaagatcg cattcatgcg tcttcacgtg 60**

**a 61**

<210> 18
<211> 110
<212> DNA
<213> synthetic sequence

<400> 18

**ctgcttcgtg catgagtacg tattctcttt acgtgactgc gtaagtaaac gtcgctgtgc 60**

**tagtgcagtc acagttgaaa ctcaagatcg cattcaagct ctttactgct 110**

<210> 19
<211> 113
<212> **DNA**
<213> synthetic sequence

<400> 19

**tcgcattcaa gctctttact gcttacgtga ctgcgttgca gtcacagctt agtacgtatt 60**

**gcgtcttcag tgcatgttga aactcaagat cgcattcatg cgtcttcacg tga 113**

<210> 20
<211> 114
<212> DNA
<213> synthetic sequence

<400> 20

**ctgcttcgtg catgagtacg tattctcttt acgtgactgc gtaagtaaac gtcgctgtgc 60**

**tagtgcagtc acagttgaaa ctcaagatcg cattcaaggt ctttactgct tacg 114**

<210> 21
<211> 112
<212> **DNA**
<213> synthetic sequence

<400> 21

**cgcattcaag gtctttactg cttacgtgac tgcgttgcag tcacagctta gtacgtattg 60**

**cgtcttcagt gcatgttgaa actcaagatc gcattcatgc gtcttcacgt ga 112**

<210> 22

<211> 110
<212> **DNA**
<213> synthetic sequence

<400> 22

**ctgcttcgtg catgcggacg tattctcttt acgtgactgc gtaagtaaac gtcgctgtgc 60**

**tagtgcagtc acagttgaaa ctcaagatcg cattcaaggt cgttactgct 110**

<210> 23
<211> 113
<212> **DNA**
<213> synthetic sequence

<400> 23

**tcgcattcaa ggtcgttact gcttacgtga ctgcgttgca gtcacagctt agtacgtatt 60**

**gcgtcttcag tgcatgttga aactcaaggt cgcattcatg cgtcttcacg tga 113**

<210> 24
<211> 50
<212> DNA
<213> synthetic sequence

<400> 24
gccctcatga tattttaaaa cacagcatcc tcaaccttga ggcggaggtc 50

<210> 25
<211> 49
<212> DNA
<213> synthetic sequence

<400> 25
ccttggcctg gcttgcttac cttgttatca agtcctgaat gggggaagc 49

<210> 26
<211> 20
<212> DNA
<213> synthetic sequence

<400> 26
ctctcccact gcatctgtca 20

<210> 27
<211> 20
<212> DNA
<213> synthetic sequence

<400> 27
catatcccca tggcaaactc 20

<210> 28
<211> 19

<212> DNA
<213> synthetic sequence

<400> 28
cacactgacg tgcctctcc 19

<210> 29
<211> 19
<212> DNA
<213> synthetic sequence

<400> 29
gcaggtactg ggagccaat 19

<210> 30
<211> 20
<212> DNA
<213> synthetic sequence

<400> 30
tctccacaag gaggcatgga 20

<210> 31
<211> 20
<212> DNA
<213> synthetic sequence

<400> 31
gacaggacca ttgcccacag 20

<210> 32
<211> 27
<212> DNA
<213> synthetic sequence

<400> 32
cgttccagaa gcggcagaag cttacct 27

<210> 33
<211> 30
<212> DNA
<213> synthetic sequence

<400> 33
acctgggttg aagcaattct ccagccttag 30

<210> 34
<211> 4145
<212> DNA
<213> synthetic sequence

<400> 34

```
catcatcatc atcatcacgg atccatggac aagaagtaca gcatcggcct ggccatcggc        60
accaactctg tgggctgggc cgtgatcacc gacgagtaca aggtgcccag caagaaattc       120
aaggtgctgg gcaacaccga ccggcacagc atcaagaaga acctgatcgg agccctgctg       180
```

ttcgacagcg gcgaaacagc cgaggccacc cggctgaaga gaaccgccag aagaagatac 240
accagacgga agaaccggat ctgctatctg caagagatct tcagcaacga gatggccaag 300
gtggacgaca gcttcttcca cagactggaa gagtccttcc tggtggaaga ggataagaag 360
cacgagcggc accccatctt cggcaacatc gtggacgagg tggcctacca cgagaagtac 420
cccaccatct accacctgag aaagaaactg gtggacagca ccgacaaggc cgacctgcgg 480
ctgatctatc tggccctggc ccacatgatc aagttccggg gccacttcct gatcgagggc 540
gacctgaacc ccgacaacag cgacgtggac aagctgttca tccagctggt gcagacctac 600
aaccagctgt tcgaggaaaa ccccatcaac gccagcggcg tggacgccaa ggccatcctg 660
tctgccagac tgagcaagag cagacggctg gaaaatctga tcgcccagct gcccggcgag 720
aagaagaatg gcctgttcgg caacctgatt gccctgagcc tgggcctgac ccccaacttc 780
aagagcaact tcgacctggc cgaggatgcc aaactgcagc tgagcaagga cacctacgac 840
gacgacctgg acaacctgct ggcccagatc ggcgaccagt acgccgacct gtttctggcc 900
gccaagaacc tgtccgacgc catcctgctg agcgacatcc tgagagtgaa caccgagatc 960
accaaggccc ccctgagcgc ctctatgatc aagagatacg acgagcacca ccaggacctg 1020
accctgctga aagctctcgt gcggcagcag ctgcctgaga agtacaaaga gattttcttc 1080
gaccagagca agaacggcta cgccggctac attgacggcg gagccagcca ggaagagttc 1140
tacaagttca tcaagcccat cctggaaaag atggacggca ccgaggaact gctcgtgaag 1200
ctgaacagag aggacctgct gcggaagcag cggaccttcg acaacggcag catcccccac 1260
cagatccacc tgggagagct gcacgccatt ctgcggcggc aggaagattt ttacccattc 1320
ctgaaggaca accgggaaaa gatcgagaag atcctgacct tccgcatccc ctactacgtg 1380
ggccctctgg ccaggggaaa cagcagattc gcctggatga ccagaaagag cgaggaaacc 1440
atcacccccct ggaacttcga ggaagtggtg gacaagggcg cttccgccca gagcttcatc 1500
gagcggatga ccaacttcga taagaacctg cccaacgaga aggtgctgcc caagcacagc 1560
ctgctgtacg agtacttcac cgtgtataac gagctgacca aagtgaaata cgtgaccgag 1620
ggaatgagaa agcccgcctt cctgagcggc gagcagaaaa aggccatcgt ggacctgctg 1680
ttcaagacca accggaaagt gaccgtgaag cagctgaaag aggactactt caagaaaatc 1740
gagtgcttcg actccgtgga aatctccggc gtggaagatc ggttcaacgc ctccctgggc 1800
acataccacg atctgctgaa aattatcaag gacaaggact tcctggacaa tgaggaaaac 1860
gaggacattc tggaagatat cgtgctgacc ctgacactgt ttgaggacag agagatgatc 1920

gaggaacggc tgaaaaccta tgcccacctg ttcgacgaca aagtgatgaa gcagctgaag 1980

cggcggagat acaccggctg gggcaggctg agccggaagc tgatcaacgg catccgggac 2040

aagcagtccg gcaagacaat cctggatttc ctgaagtccg acggcttcgc caacagaaac 2100

ttcatgcagc tgatccacga cgacagcctg acctttaaag aggacatcca gaaagcccag 2160

gtgtccggcc agggcgatag cctgcacgag cacattgcca atctggccgg cagccccgcc 2220

attaagaagg gcatcctgca gacagtgaag gtggtggacg agctcgtgaa agtgatgggc 2280

cggcacaagc ccgagaacat cgtgatcgaa atggccagag agaaccagac cacccagaag 2340

ggacagaaga acagccgcga gagaatgaag cggatcgaag agggcatcaa agagctgggc 2400

agccagatcc tgaaagaaca ccccgtggaa aacacccagc tgcagaacga gaagctgtac 2460

ctgtactacc tgcagaatgg gcgggatatg tacgtggacc aggaactgga catcaaccgg 2520

ctgtccgact acgatgtgga ccacatcgtg cctcagagct ttctgaagga cgactccatc 2580

gacaacaagg tgctgaccag aagcgacaag gcccggggca agagcgacaa cgtgccctcc 2640

gaagaggtcg tgaagaagat gaagaactac tggcggcagc tgctgaacgc caagctgatt 2700

acccagagaa agttcgacaa tctgaccaag gccgagagag gcggcctgag cgaactggat 2760

aaggccggct tcatcaagag acagctggtg gaaacccggc agatcacaaa gcacgtggca 2820

cagatcctgg actcccggat gaacactaag tacgacgaga atgacaagct gatccgggaa 2880

gtgaaagtga tcaccctgaa gtccaagctg gtgtccgatt tccggaagga tttccagttt 2940

tacaaagtgc gcgagatcaa caactaccac cacgcccacg acgcctacct gaacgccgtc 3000

gtgggaaccg ccctgatcaa aaagtaccct aagctggaaa gcgagttcgt gtacggcgac 3060

tacaaggtgt acgacgtgcg gaagatgatc gccaagagcg agcaggaaat cggcaaggct 3120

accgccaagt acttcttcta cagcaacatc atgaactttt tcaagaccga gattaccctg 3180

gccaacggcg agatccggaa gcggcctctg atcgagacaa acggcgaaac cggggagatc 3240

gtgtgggata agggccggga ttttgccacc gtgcggaaag tgctgagcat gccccaagtg 3300

aatatcgtga aaaagaccga ggtgcagaca ggcggcttca gcaaagagtc tatcctgccc 3360

aagaggaaca gcgataagct gatcgccaga aagaaggact gggaccctaa gaagtacggc 3420

ggcttcgaca gccccaccgt ggcctattct gtgctggtgg tggccaaagt ggaaaagggc 3480

aagtccaaga aactgaagag tgtgaaagag ctgctgggga tcaccatcat ggaaagaagc 3540

agcttcgaga agaatcccat cgactttctg gaagccaagg tgaaccccga caacagctga 3600

tcatcaagct gcctaagtac tccctgttcg agctggaaaa cggccggaag agaatgctgg 3660

```
cctctgccgg cgaactgcag aagggaaacg aactggccct gccctccaaa tatgtgaact     3720
tcctgtacct ggccagccac tatgagaagc tgaagggctc ccccgaggat aatgagcaga     3780
aacagctgtt tgtggaacag cacaagcact acctggacga gatcatcgag cagatcagcg     3840
agttctccaa gagagtgatc ctggccgacg ctaatctgga caaagtgctg tccgcctaca     3900
acaagcaccg ggataagccc atcagagagc aggccgagaa tatcatccac ctgtttaccc     3960
tgaccaatct gggagcccct gccgccttca agtactttga caccaccatc gaccggaaga     4020
ggtacaccag caccaaagag gtgctggacg ccaccctgat ccaccagagc atcaccggcc     4080
tgtacgagac acggatcgac ctgtctcagc tgggaggcga cgattacaag gatgacgatg     4140
acaag                                                                 4145
```